# EUROPEAN PATENT SPECIFICATION

(11) **EP 3 392 337 B1**
(45) Date of publication and mention of the grant of the patent: **06.03.2024**
(21) Application number: 16875388.7
(22) Date of filing: 29.11.2016
(51) Int. Cl.: C12N 15/09, A01K 67/027, C12N 5/16, C12N 9/22, C12N 15/113, C12N 15/90

(54) **GENETIC MODIFICATION NON-HUMAN ORGANISM, EGG CELLS, FERTILIZED EGGS, AND METHOD FOR MODIFYING TARGET GENES**
GENETISCHE VERÄNDERUNG EINES NICHT-MENSCHLICHEN ORGANISMUS, EIZELLEN, BEFRUCHTETE EIZELLEN UND VERFAHREN ZUR MODIFIZIERUNG VON ZIELGENEN
ORGANISME NON-HUMAIN GÉNÉTIQUEMENT MODIFIÉ, OVOTIDE, OEUF FÉCONDÉ, ET PROCÉDÉ DE MODIFICATION DE GÈNE CIBLE

(30) Priority: 18.12.2015 JP 2015247960
(43) Date of publication of application: 24.10.2018
(73) Proprietor: Japan Science and Technology Agency, Kawaguchi-shi, Saitama 332-0012 (JP)
(72) Inventor: SHINDO, Takayuki, Matsumoto-shi Nagano 390-8621 (JP); SAKURAI, Takayuki, Matsumoto-shi Nagano 390-8621 (JP)
(74) Representative: Plasseraud IP
(86) International application number: PCT/JP2016/085391
(87) International publication number: WO 2017/104404

(56) References cited:
- WO-A1-2013/176772
- WO-A1-2014/204725
- WO-A1-2014/204725
- WO-A1-2015/148761
- WO-A1-2016/080097
- MIKAMI MASAFUMI ET AL: "Parameters affecting frequency of CRISPR/Cas9 mediated targeted mutagenesis in rice", PLANT CELL REPORTS, SPRINGER, BERLIN/HEIDELBERG, vol. 34, no. 10, 2 July 2015 (2015-07-02), pages 1807-1815, XP035546825, ISSN: 0721-7714, DOI: 10.1007/S00299-015-1826-5 [retrieved on 2015-07-02]
- DAISUKE MASHIKO ET AL: "Generation of mutant mice by pronuclear injection of circular plasmid expressing Cas9 and single guided RNA", SCIENTIFIC REPORTS, vol. 3, no. 1, 27 November 2013 (2013-11-27), XP055391490, DOI: 10.1038/srep03355
- TANG HAI ET AL: "One-step generation of knockout pigs by zygote injection of CRISPR/Cas system", CELL RESEARCH - XIBAO YANJIU, vol. 24, no. 3, 31 January 2014 (2014-01-31), pages 372-375, XP055227259, GB, CN ISSN: 1001-0602, DOI: 10.1038/cr.2014.11
- Anonymous: "CRISPR Knockout Screens with Optimal Cas9 to sgRNA Ratios - Cellecta, Inc", , 2 May 2015 (2015-05-02), XP055587567, Retrieved from the Internet: URL:https://web.archive.org/web/2015050212 4654/https://www.cellecta.com/crispr-knock out-screens-with-optimal-cas9-to-sgrna-rat ios/ [retrieved on 2019-05-10]
- TAKAYUKI SAKURAI ET AL: "A non-inheritable maternal Cas9-based multiple-gene editing system in mice", SCIENTIFIC REPORTS, vol. 6, no. 1, 28 January 2016 (2016-01-28), XP055523965, DOI: 10.1038/srep20011
- KYEONG-JUN U et al.: "Generation of Cas9 transgenic mice and its application", Transgenic Research, vol. 23, no. 5, 2014, pages 871-872, XP009511054,
- YIN L et al.: "Multiplex conditional mutagenesis using transgenic expression of Cas9 and sgRNAs", Genetics, vol. 200, no. 2, June 2015 (2015-06), pages 431-441, XP009189703,
- MASHIKO D et al.: "Generation of mutant mice by pronuclear injection of circular plasmid expressing Cas9 and single guided RNA", Scientific Reports, vol. 3, no. 1, 27 November 2013 (2013-11-27), page 3355, XP055391490,
- PLATT RJ et al.: "CRISPR-Cas9 knockin mice for genome editing and cancer modeling", Cell Press, vol. 159, no. 2, 25 September 2014 (2014-09-25), pages 440-455, XP029073412,
- CARROLL KJ et al.: "A mouse model for adult cardiac-specific gene deletion with CRISPR/Cas9", Proceedings of the National Academy of Sciences of the United States of America, vol. 113, no. 2, 12 January 2016 (2016-01-12), pages 338-343, XP055391492,

## Description

### Technical Field

The present invention relates to female reproductive cells derived from a genetically modified non-human mammal characterized in that a protein or mRNA of maternally derived Cas9 accumulates in the female reproductive cells, wherein the genetically modified non-human mammal has a nuclear genome into which at least 3 copies of genes that code for Cas9 (CRISPR-associated 9) are introduced and wherein the genetically modified non-human mammal is one selected from the group consisting of a mouse, rat, hamster, guinea pig, rabbit, dog, cat, horse, cow, sheep, pig, goat, marmoset, and monkey..

Priority is claimed on Japanese Patent Application No. 2015-247960, filed on December 18, 2015.

### Background Art

In research on disease mechanisms and development research for a new drug, the existence of an animal disease model bearing pathologies of the disease holds the key to the success of the research. In regard to genetic factors of a human disease, it has become possible to relatively easily obtain genetic information for several million locations and an SNP locus with genome analysis techniques undergoing rapid development in recent years. Many loci of disease-associated genes have been identified and reported for many diseases using the technique. On the other hand, even in a case where genetic modification mice having such disease candidate genes are produced, a phenotype is not exhibited in most cases. This is because an abnormality in a single gene does not necessarily explain pathologies in which multiple factors are involved, such as lifestyle diseases. Accordingly, it is considered that an effect of drug development targeting each of these candidate genes is limited. In order to solve such problems, there is a need to prepare a system to quickly and systematically prepare and utilize an animal disease model of multilocus modification, but in a method for genetic modification by ES cells of the related art, only genetic modification of a single operation, one locus, and a gene at one location was possible, and therefore it was difficult to realize the above solution.

Recently, a genetic modification technique by CRISPR (Clustered Regularly Interspaced Short Palindromic Repeats) system has appeared. The CRISPR system has the potential of a novelty that transcends the ES cell-mediated genetic modification technique of the related art. In the CRISPR system, three types of RNAs or DNAs, which are a guide RNA that guides cleavage of a target gene, an mRNA of Cas9 (CRISPR-associated 9) which is a nuclease actually involved in the cleavage, and a modified gene that is homologously inserted into a gene region to be cleaved, are microinjected into a fertilized egg so as to develop the embryo thereof up to the adult cell, and therefore genetic modification mice can be obtained.

It is disclosed in NPL 1 that by using the CRISPR system, knockin of genes that code for Cas9 into a ROSA26 gene region of ES cells is performed, and thus Cas9 knockin mice can be produced.

### Citation List

### Patent Literature

[NPL 1]: Randall J. Platt, et al., Cell, vol. 159, p. 440-455, 2014

### Summary of Invention

### Technical Problem

In the method for producing genetic modification mice utilizing the CRISPR system of the related art, the number of mRNA of Cas9 was large, and therefore amounts of other guide RNAs to be microinjected and modified genes were limited.

In addition, in the Cas9 knockin mice disclosed in NPL 1, because only one copy of the gene of Cas9 has been introduced, it was difficult to edit a plurality of different genes or a plurality of different locations in the same gene at the same time.

The present invention has been made in view of the above circumstances, and an object thereof is to provide a genetic modification non-human organism in which an expression level of Cas9 is high and a plurality of different genes or a plurality of different locations in the same gene can be edited at the same time with high efficiency. The present invention further provides a genetic modification technique which is simply and rapidly and site-specifically performed with respect to a target gene using the genetic modification non-human organism.

### Solution to Problem

The present invention relates to female reproductive cells derived from a genetically modified non-human mammal characterized in that a protein or mRNA of maternally derived Cas9 accumulates in the female reproductive cells, wherein the genetically modified non-human mammal has a nuclear genome into which at least 3 copies of genes that code for Cas9 (CRISPR-associated 9) are introduced and wherein the genetically modified non-human mammal is one selected from the group consisting of a mouse, rat, hamster, guinea pig, rabbit, dog, cat, horse, cow, sheep, pig, goat, marmoset, and monkey.
The invention is as defined in claims 1 to 6.
That is, the present invention includes the following aspects.
[1] Egg cells or female reproductive cells derived from a genetic modification non-human organism having a nuclear genome into which at least 3 copies of genes that code for Cas9 are introduced, wherein the genetically modified non-human mammal is one selected from the group consisting of a mouse, rat, hamster, guinea pig, rabbit, dog, cat, horse, cow, sheep, pig, goat, marmoset, and monkey
[2] The egg cells according to [2], in which in the nucleus of the egg cell, the nuclear genome into which the genes that code for the Cas9 are introduced is not contained.
[3] A method for modifying target genes, including a step of introducing a guide RNA into the egg cells according to [1] or [2].
[4] A method for modifying target genes, including:
   a step of introducing a guide RNA into the egg cells according to [1] or [2];
   a step of cleaving the target genes at a cleavage site located upstream of a PAM (proto-spacer adjacent motif) sequence by Cas9 expressed in the egg cells derived from the genetic modification non-human organism; and
   a step of obtaining the modified target genes in a region determined by complementary binding of the guide RNA and the target genes,
   in which the target genes have the PAM sequence, and
   the guide RNA contains a polynucleotide including a base sequence complementary to an upstream base sequence of the PAM sequence in the target genes.
[5] A method for modifying target genes, including:
   a step of introducing a guide RNA into the egg cells according to [1] or [2];
   a step of cleaving the target genes at a cleavage site located three bases upstream of a PAM sequence by Cas9 expressed in the egg cells derived from the genetic modification non-human organism; and
   a step of obtaining the modified target genes in a region determined by complementary binding of the guide RNA and the target genes,
   in which the target genes have the PAM sequence including NGG (N is any one base selected from the group consisting of adenine, cytosine, thymine, and guanine), and
   the guide RNA contains a polynucleotide including base sequences complementary to base sequences from one base upstream to 20 bases to 24 bases upstream of the PAM sequence in the target genes.
[6] The method for modifying target genes according to any one of [3] to [5], in which in the introduction step, two or more of the guide RNAs are introduced.
[7] The method for modifying target genes according to any one of [3] to [6], in which in the introduction step, a foreign gene is introduced together with the guide RNAs.
[8] The method for modifying target genes according to [17], in which the foreign gene does not have the PAM sequence.

### Advantageous Effects of Invention

According to the genetic modification non-human organism, of the present invention, a plurality of different genes or a plurality of different locations in the same gene can be edited at the same time with high efficiency. Furthermore, the genetic modification non-human organism of the present invention can be utilized in studies of lifestyle diseases such as cancer or heart disease in which abnormality of many genes is involved, or disease-related genes identified by genome-wide association study (GWAS).

### Brief Description of Drawings

FIG. 1A is a schematic diagram showing each structure of NFCas9 (Cas9 gene having a NLS and a Flag tag) and FCas9 (Cas9 having only the Flag tag) in Example 1.
FIG. 1B is a graph comparing the copy number of Cas9 mRNA in 50 ng RNA in each tissue of a mouse of NFCas9-2 line in Test Example 1.
FIG. 1C is a graph showing changes in body weight of male or female mice (4 weeks old to 10 weeks old) having different genotypes of NFCas9-2 line in Test Example 1.
FIG. 1D shows images of results in which each tissue section of mice (10 weeks old) having different genotypes of NFCas9-2 line is stained with hematoxylin and eosin in Test Example 1.
FIG. 1E is a graph showing results of detecting an amount of a physiological marker in serum of the mouse (10 weeks old) having different genotypes of NFCas9-2 line in Test Example 1.
FIG. 2A is a table showing the copy number of Cas9 genes introduced in 4 lines of NFCas9 mouse and 5 lines of FCas9 mouse constructed in Example 1, sex, a genotype in F1 generation, presence or absence of Cas9 transmission into F1 generation, and an expression level of Cas9.
FIG. 2B is a graph showing a calibration curve for calculating the copy number of Cas9 introduced in 4 lines of NFCas9 mouse and 5 lines of FCas9 mouse constructed in Example 1.
FIG. 2C shows images of results of performing a western blot analysis using Flag tag on expression of Cas9 protein NACas9 mouse and FCas9 mouse constructed in Example 1.
FIG. 3A is an image showing results in which PCR products of SpPCR are detected by 1% agarose gel electrophoresis using genomic DNA of NFCas9-2 line in Example 1.
FIG. 3B is a schematic diagram showing a location where NFCas9 of NFCas9-2 line in Example 1 is inserted.
FIG. 3C is an image showing results in which PCR products obtained by a site-specific primer for confirming the results in FIG. 3B in Example 1 are detected by 1% agarose gel electrophoresis.
FIG. 4A is a diagram showing genotypes in fertilized eggs obtained by artificial fertilization of various crossing pairs in Test Example 2.
FIG. 4B is a table showing the number of viable eggs and the number of indel mutations in the fertilized eggs obtained by artificial fertilization of various crossing pairs in Test Example 2.
FIG. 4C is a schematic diagram showing a sequence of a guide RNA used for modification of Ramp2 gene in Test Example 2.
FIG. 4D is an image showing results of T7 endonuclease I (T7EI) assay in Test Example 2.
FIG. 5A is a table showing the number of viable zygotes and the number of indel mutations in Test Example 3.
FIG. 5B shows images of results of T7EI assay of the blastocysts in Test Example 3.
FIG. 6A is a table showing the number of viable eggs and the number of indel mutated embryos after 13.5 days to 15.5 days from implantation in Test Example 3.
FIG. 6B shows images of forms of embryos of ten pups after 13.5 days from implantation in Test Example 3.
FIG. 6C shows images of results of T7EI assay on blastocysts of the embryos after 13.5 days from implantation in Test Example 3.
FIG. 6D is a diagram showing results of sequences of Ramp 1 gene of the embryos of ten pups after 13.5 days from implantation in Test Example 3.
FIG. 6E is a diagram showing relative RNA expression levels in Ramp1 gene and Ramp2 gene of the embryos of ten pups after 13.5 days from implantation in Test Example 3.
FIG. 7A shows a table and a graph showing determination results of genotypes and the presence or absence of mutations in Test Example 4.
FIG. 7B is an image showing results of T7EI assay of the blastocysts in Test Example 4.
FIG. 8 is a schematic diagram showing sequences of guide RNAs used for modification of genes in Test Example 5.
FIG. 9A is a table showing determination results of genotypes and the presence or absence of mutations in Test Example 5 in a case of using a method of the present invention.
FIG. 9B shows images of results of T7EI assay on blastocysts of ten embryos in Test Example 5 by using the method of the present invention.
FIG. 9C is a table showing determination results of genotypes and the presence or absence of mutations in Test Example 5 in a case of using a method of the related art as a control group.
FIG. 10A is a schematic diagram showing a protocol in Test Example 6.
FIG. 10B is a schematic diagram showing a sequence of a guide RNA used for modification of Ggat1 gene in Test Example 6.
FIG. 10C shows images of results of T7EI assay on fibroblasts in Test Example 6.
FIG. 10D is a graph showing results of FACS analysis utilizing the fact that FITC-labeled isolectin, BS-I-B4 (IB4) specifically recognizes α-Gal epitope in Test Example 6.
FIG. 10E is an image in which fibroblasts are imaged after a survival assay by treatment with IB4-saporin conjugate (IB4SAP) in Test Example 6.
FIG. 11A is a schematic diagram showing a protocol in Test Example 7.
FIG. 11B is a schematic diagram showing a sequence of a guide RNA used for modification of Alb1 gene in Test Example 7.
FIG. 11C is an image showing results of T7EI assay on EGFP-positive or EGFP-negative hepatocytes of a Cas9 mouse or a wild-type mouse in Test Example 7.
FIG. 12A is a table showing indel mutation ratios of 12.5- to 13-day-old fetuses in Test Example 8.
FIG. 12B is an image showing the jaw formation state of 12.5- to 13-day-old endothelin 1 (ET-1) gene knockout phenotype mouse fetus and wild-type mouse fetus in Test Example 8.
FIG. 12C is a graph showing mosaic ratios of fetuses derived from each fertilized egg in Test Example 8.
FIG. 13 is a graph showing mosaic ratios of fetuses derived from each of fertilized eggs having different introduction times of a guide RNA in Test Example 9.
FIG. 14 is a graph showing indel mutation ratios and mosaic ratios of fetuses derived from each of fertilized eggs having different introduction concentrations of a guide RNA in Test Example 10.
FIG. 15A is a schematic diagram showing a sequence of a knock-in fragment and a guide RNA used for modification of genes in Test Example 11.
FIG. 15B is an image showing a result of subjecting a PCR product obtained by using Klf5 gene as a template in PCR, to agarose gel electrophoresis by using a solution prepared from a blastocyst derived from a fertilized egg of a Cas9 mouse in Test Example 11.
FIG. 15C is a table showing knockin efficiency in a blastocyst derived from a fertilized egg of a Cas9 mouse or a wild-type mouse in Test Example 11.

### Description of Embodiments

### <Genetic Modification Non-Human Organism>

As previously mentioned, it is hereby described a genetic modification non-human organism in which a nuclear genome includes at least 3 copies of genes that code for Cas9 (CRISPR-associated 9), which is not part of the invention.

In the genetic modification non-human organism, Cas9 is overexpressed in all or any one of cells. According to the inventors of the present invention, it became clear that an overexpressed state of Cas9 does not affect the body of the non-human organism. In addition, because Cas9 is overexpressed in advance, it is possible to simply and rapidly modify target genes by only introducing a guide RNA.

In the present specification, the term "genetic modification" means performing of specific gene disruption, knockin of a reporter gene, and the like by carrying out recombination of targeted gene or targeted mutation (for example, insertion of SNP, partial mutation, and the like) through techniques such as CRISPR/Cas9 system, Transcription Activator-Like Effector Nucleases (TALEN), and the like.

In the present specification, examples of the "non-human organism" include, but are not limited to, E. coli, Bacillus subtilis, yeast, insect, plant (such as monocotyledon or dicotyledon), bird animals such as chickens, mammals such as mice, rats, hamsters, guinea pigs, rabbits, dogs, cats, horses, cows, sheep, pigs, goats, marmosets, and monkeys. Among these, mammals are preferable, and rodents such as mice, rats, guinea pigs, hamsters, and rabbits are preferable.

Examples of mice include C57BL/6 strain, DBA2 strain, and the like as pure strains, include B6C3F1 strain, BDF1 strain, B6D2F1 strain, BALB/c strain, ICR strain, and the like as hybrid strains, but the examples are not limited thereto. Specific examples of rats include Wistar, SD, and the like, but the examples are not limited thereto.

### [Cas9]

"Cas9" is one of the Cas protein families constituting the adaptive immune system that provides acquisition resistance against invading foreign nucleic acid in bacteria and archaebacteria and is an endonuclease that recognizes a PAM sequence in exogenous invading DNA so as to cleave the double-stranded DNA to produce upstream blunt ends. In the present specification, Cas9 means an enzyme that forms a complex with the guide RNA and has DNA cleavage activity.

In the present specification, examples of Cas protein families include Cas1, Cas1B, Cas2, Cas3, Cas4, Cas5, Cas6, Cas7, Cas8, Cas9 (known as Csnl and Csx12), Cas 10, Csy1, Csy2, Csy3, Csel, Cse2, Cscl, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, modified ones thereof, and the like. Among these, it is particularly preferable that Cas9 genes, homologues thereof, or modified ones thereof be introduced into the genetic modification non-human organism.

Cas9 directs cleavage of one or both strands within base pairs of, for example, 1, 2, 3, 4, 5, 6, 7, 8, 9, 10, 15, 20, 25, 50, 100, 200, 500 or more, from the first or last nucleotide of the PAM sequence in a target gene. Which of bp upsteam of the PAM sequence is to be cleaved depends on bacterial species derived from Cas9, but most of Cas9 including the one from Streptococcus pyogenes (S. pyogenes) cleaves three bases upstream of the PAM sequence.

In addition, Cas9 used may be a variant lacking activity of cleaving one or both strands of a target gene containing a target sequence. For example, substitution from aspartic acid in a RuvCI catalytic domain of Cas9 derived from S. pyogenes to alanine (D10A) converts Cas9 from nuclease which cleaves both strands to nickase (which cleaves one strand). Other examples of mutations in which Cas9 is converted to nickase include, but are not limited to, H840A, N854A, and N863A. In addition, two or more catalytic domains of Cas9 (RuvCI, RuvCII, and RuvCIII) can produce mutated Cas9 in which entire DNA cleavage activity is substantially lost. In this case, Cas9 is preferably other chimeric enzymes fused with an enzyme having DNA cleavage activity. The D10A mutation may be combined with one or a plurality of H840A, N854A, or N863A mutations so as to produce the Cas9 in which entire DNA cleavage activity is substantially lost. In a case where the DNA cleavage activity of the mutated Cas9 is less than about 25%, 10%, 5%, 1%, 0.1%, or 0.01% with respect to a non-mutated form thereof, entire DNA cleavage activity is regarded to be substantially lost.

A base sequence that codes for Ca9 may be codon-optimized for expression in specific cells such as eukaryotic cells. Examples of eukaryotic cells include, but are not limited to, specific organisms such as humans, mice, rats, rabbits, dogs, pigs, or nonhuman primates. In general, codon optimization refers to a process in which while maintaining native amino acid sequences, at least one codon of the native sequence is replaced with a codon that is used more frequently or most frequently in organism genes to be introduced, and thus a nucleic acid sequence is modified for enhanced expression in the organism genes to be introduced. Various species exhibit a specific bias for a specific codon of a specific amino acid. The codon bias (difference in frequency of occurrence of codon usage between organisms) is often correlated with the translation efficiency of mRNA, and it is considered that this depends especially on characteristics of the codon being translated and the availability of a specific tRNA. The predominant selected tRNAs in cells is generally a reflection of the most frequently used codons in peptide synthesis. Accordingly, genes can be personalized for optimal gene expression in a predetermined organism based on codon optimization. The frequency of occurrence of codon usage is readily available in the "Codon Usage Database" posted on www.kazusa.or.jp/codon/ (visited on December 16, 2015), and by using this table, it is possible to optimize the codon (for example, refer to "Codon usage tabulated from the international DNA sequence databases: status for the year 2000", Nucl. Acids Res. 28:292 (2000) by Nakamura, Y, et al. Computer algorithms for codon optimization of specific sequences for expression in a specific organism species are also available, for example, in Gene Forge (Aptagen, LLC.; Jacobus, PA), and the like. One or a plurality of codons in the sequence that codes for Cas9 corresponds to the codon most frequently used for a specific amino acid.

As an expression level of Cas9 cells increases, it is not necessary to introduce Cas9 from the outside, and therefore a large amount of guide RNA can be introduced. Therefore, it is possible to efficiently carry out genetic modification on one location or a plurality of locations with respect to one gene by using the CRISPR system, and it is also possible to modify not only one gene but also a plurality of genes at the same time by a single operation. It is preferable that the copy number of the Cas9 gene introduced be 3 or more. This is because that in general, as the copy number of the introduced Cas9 gene increases, an expression level of Cas9 tends to increase, although an expression level is also influenced by a locus into which the Cas9 gene is introduced, and a promoter. The copy number of a gene that codes for the introduced Cas9 may be 3, 4, 5, 6, 7, 8, 9, 10, 20, 50 or more, for example.

In the present specification, the term "nuclear genome" means all chromosomes contained in the nucleus. For example, in a case of humans, the number of chromosomes constituting the nuclear genome is a total 46 of 22 pairs of autosomal chromosomes and two sex chromosomes. In a case of bovines, the number of chromosomes is a total of 60 of 29 pairs of autosomal chromosomes and two sex chromosomes. In a case of mice, the number of chromosomes is a total of 40 of 19 pairs of autosomal chromosomes and two sex chromosomes.

In the present specification, the term "copy number" means the number of base sequences of a specific gene present in the nuclear genome. For example, in a case where one specific gene is present on one chromosome constituting the nuclear genome, the copy number is 1. In addition, in a case where one specific gene is present on one chromosome and a homologous chromosome thereof (that is, in a case of a homozygote), the copy number is 2.

In the present embodiment, a plurality of Cas9 genes may be introduced into the same chromosome, and one or a plurality of Cas9 genes may be introduced into different chromosomes. In addition, the Cas9 gene may be tandemly introduced into the same locus on the same chromosome.

In the present embodiment, a locus into which the Cas9 gene is introduced is a gene region in which the expression is constantly and stably performed and is a region that enables the survival of life even in a case where a gene originally coded in this region is lost or modified. In addition, in a case of utilizing the CRISPR system in a case where the Cas9 gene is introduced, it is preferable to have a PAM sequence in the vicinity. Examples of the locus into which the Cas9 gene is introduced include GTP-binding protein 10 locus, Rosa26 locus, beta-actin locus, and the like.

### [Promoter]

In the present embodiment, the Cas9 gene introduced into the nuclear genome may contain a promoter operably linked to the Cas9 gene. Examples of the promoter include a viral promoter, an expression-inducible promoter, a tissue-specific promoter, or a promoter obtained by fusing an enhancer sequence or a promoter sequence, and the like. It is preferable that the promoter be linked to upstream (5' side) of the Cas9 gene.

In the present specification, the term "viral promoter" means a virus-derived promoter. Examples of viruses from which a promoter is derived include cytomegalovirus, moloney leukemia virus, JC virus, mammary tumor virus, simian virus, retrovirus, and the like.

In the present specification, the term "expression-inducible promoter" means a promoter by which the expression of a gene (in the present embodiment, the Cas9 gene) to be expressed in a case where a specific stimulus such as a chemical agent or physical stress is given is caused and expression activity is not caused in the absence of stimulation. Examples of the expression-inducible promoter include, but not are limited to, a TetO (tetracycline operator) promoter, a metallothionein promoter, IPTG/lacI promoter system, ecdysone promoter system, "lox stop lox" system by which an inhibitory sequence is irreversibly lost during translation or transcription, and the like.

In the present specification, the term "tissue-specific promoter" means a promoter which exhibits activity only in a specific tissue. Examples of the tissue-specific promoter include, but are not limited to, a tyrosinase promoter or a TRP2 promoter in a case of melanoma cells or melanocytes; a MMTV promoter or a WAP promoter in a case of breast cells or breast cancer; a villin promoter or an FABP promoter in a case of intestinal cells or intestinal cancer; a PDX promoter in a case of pancreatic cells; a RIP promoter in a case of pancreatic beta cells; a keratin promoter in a case of keratinocytes; a probasin promoter in a case of prostate epithelium; a nestin promoter or a GFAP promoter in a case of cells of the central nervous system (CNS) or cancers of the central nervous system; a tyrosine hydroxylase promoter, an S100 promoter, or a neurofilament promoter in a case of neurons; a pancreas-specific promoter described in Edlund et al., Science, 230:912-916 (1985); a clara cell secretory protein promoter in a case of lung cancer; an α-myosin promoter in a case of cardiac cells, and the like.

Examples of a promoter obtained by fusing an enhancer sequence or a promoter sequence include an SR-α promoter including a promoter of early genes of simian virus 40 (SV40) and a partial sequence of the long terminal repeat of human T-cell leukemia virus 1; a CAG promoter including immediate early (IE) gene enhancer of cytomegalovirus and a chicken β-actin promoter; and the like. In particular, the CAG promoter enables overexpression of a gene to be expressed (in the present embodiment, the Cas9 gene) in almost the whole body by providing a poly(A) signal site of a rabbit β-globin gene at the 3' end of the gene to be expressed (in the present embodiment, the Cas9 gene).

Among these, the viral promoter or the CAG promoter is preferable as a promoter in a case where the gene is desired to be constantly expressed at a high level in the whole body. In addition, in a case where a timing of expression of Cas9 or a tissue to be expressed is adjusted, the expression-inducible promoter or the tissue-specific promoter is preferable.

In the present specification, the phrase "operably linked" means a functional link between a gene expression regulatory sequence (for example, a promoter or a series of transcription factor binding sites) and a gene to be expressed (in the present embodiment, the Cas9 gene). The term "expression regulatory sequence" means a sequence which directs transcription of a gene to be expressed (in the present embodiment, the Cas9 gene).

The Cas9 gene introduced into the nuclear genome may be linked to the downstream (3' side) of the Cas9 gene such that a polyadenylation signal necessary for polyadenylation of the 3' end of mRNA is operable. As the polyadenylation signal, there is a polyadenylation signal contained in each gene derived from the above viruses or from various human or non-human organisms, for example, a polyadenylation signal of late genes or early genes of SV40, rabbit β-globin genes, bovine growth hormone genes, human A3 adenosine receptor gene, and the like. In addition, in order to further express the Cas9 gene at a higher level, a splicing signal, an enhancer region, a part of introns of each gene may be linked to the 5' upstream of a promoter region, between the promoter region and a translation region, or to the 3' downstream of the translation region.

In the Cas9 gene introduced into the nuclear genome, one or a plurality of nuclear localization sequences (NLS) may be operably linked to the upstream (5' side) or the downstream (3' side) of the Cas9 gene.

Examples of the NLS include, but are not limited to, NLS of the SV40 virus large T antigen having an amino acid sequence PKKKRKV (SEQ ID NO: 1); NLS derived from nucleoplasmin (for example, bisect NLS of nucleoplasmin having a sequence KRPAATKKAGQAKKKK (SEQ ID NO: 2)); c-myc NLS having an amino acid sequence PAAKRVKLD (SEQ ID NO: 3) or RQRRNELKRSP (SEQ ID NO: 4); hRNPA1 M9 NLS having a sequence NQSSNFGPMKGGNFGGRSSGPYGGGGQYFAKPRNQGGY (SEQ ID NO: 5); a sequence RMRIZFKNKGKDTAELRRRRVEVSVELRKAKKDEQILKRRNV (SEQ ID NO: 6) of the IBB domain of importin-alpha; sequences VSRKRPRP (SEQ ID NO: 7) and PPKKARED (SEQ ID NO: 8) of fibroid T protein; a sequence PQPKKKPL (SEQ ID NO: 9) of human p53; a sequence SALIKKKKKMAP (SEQ ID NO: 10) of mouse c-abl IV; sequences DRLRR (SEQ ID NO: 11) and PKQKKR (SEQ ID NO: 12) of influenza virus NS1; a sequence RKLKKKIKKL (SEQ ID NO: 13) of hepatitis virus delta antigen; a sequence REKKKFLKRR (SEQ ID NO: 14) of mouse Mx1 protein; a sequence KRKGDEVDGVDEVAKKKSKK (SEQ ID NO: 15) of human poly(ADP-ribose) polymerase; a sequence RKCLQAGMNLEARKTKK (SEQ ID NO: 16) of steroid hormone receptor (human), glucocorticoid receptor; and the like.

In the Cas9 gene introduced into the nuclear genome, one or a plurality of marker genes such as fluorescent protein may be operably linked to the upstream (5' side) or the downstream (3' side) of the Cas9 gene. Examples of the fluorescent protein include green fluorescent protein (GFP), yellow fluorescent protein (YFP), blue fluorescent protein (BFP), cyan fluorescent protein, red fluorescent protein, and the like.

### [Method for Producing Genetic Modification Non-Human Organism]

It is also disclosed hereby a method for producing genetic modification non-human organism which is not part of the invention. The genetic modification non-human organism can be prepared by using a known genetic modification technique (for example, CRISPR system, a method of using Transcription Activator-Like Effector Nucleases (TALEN), a method of using zinc finger nuclease, and the like) for producing a genetic modification non-human organism in which foreign genes are introduced into the nuclear genome. In addition, the genetic modification non-human organism can be prepared by a method in which the Cas9 gene (preferably, one in a linear state) is introduced into a fertilized egg, an embryonic stem cell, a sperm or an unfertilized egg of a target non-human mammal, and from individuals generated using these cells, an individual in which the Cas9 gene is integrated into the chromosome of all cells containing germinal cells is selected. Among these, the genetic modification non-human organism can be efficiently obtained by a method for producing a genetic modification non-human organism using the CRISPR system of the related art. More specifically, there is a method described in "Method for Selectively and Site-Specifically Modifying Target Genes" to be described later, and the genetic modification non-human organism can be produced by using a vector containing, as a foreign gene, a gene having a sequence homologous to a locus in which a target gene is inserted into the upstream (5' side) and the downstream (3' side) of the Cas9 gene, and the Cas9 gene described above, thereby introducing the gene into the target non-human organism.

In addition, the genetic modification non-human organism may be a genetic modification non-human organism having a nuclear genome into which a foreign gene other than Cas9 gene is introduced at the same time and which thus becomes the nuclear genome into which the Cas9 gene and the foreign gene other than the Cas9 gene are introduced.

The presence of the Cas9 gene in germinal cells of the obtained genetic modification non-human organism can be confirmed by the fact that the offspring of the obtained organism has transgenes in all of the germinal cells and somatic cells thereof. Selection of individuals can be carried out by confirming at the DNA level that the Cas9 gene is present in genomic DNA prepared from some of tissues constituting the individual such as blood tissue, epithelial tissue, connective tissue, cartilage tissue, bone tissue, muscle tissue, oral tissue, or skeletal tissue.

Alternatively, the presence of the Cas9 gene can be confirmed by a genotyping method using a single blastocyst after the introduction of the Cas9 gene (reference material: Sakurai T., et al., "A single blastocyst assay optimized for detecting CRISPR/Cas9 system-induced indel mutations in mice", BMC Biotech., 14:69, 2014.). According to this method, whether or not the Cas9 gene is present can be determined more quickly compared to that of the related art.

The individual selected in the above manner is generally a heterozygote having the Cas9 gene on one side of the homologous chromosome, and thus it is possible to obtain a homozygous organism having the Cas9 gene on both sides of the homologous chromosome from the offspring by crossing individuals of the heterozygote. By crossing male with female organisms of this homozygote, all offspring become homozygotes that stably hold the Cas9 gene, and therefore transgenerational reproduction of the genetic modification non-human organism of the present embodiment is possible under general breeding environments.

### <Egg Cell>

In one embodiment, the present invention provides an egg cell or female reproductive cells derived from the genetic modification non-human organism having a nuclear genome into which at least 3 copies of the genes that code for Cas9 are introduced, wherein the genetically modified non-human mammal is one selected from the group consisting of a mouse, rat, hamster, guinea pig, rabbit, dog, cat, horse, cow, sheep, pig, goat, marmoset, and monkey.

In the egg cell of the present embodiment, a plurality of different genes or a plurality of locations in the same gene can be edited at the same time with high efficiency. In addition, according to a fertilized egg obtained by fertilizing the egg cell of the present embodiment and sperm derived from the same species of the organism, it is possible to rapidly produce the genetic modification non-human organism at a low cost.

The egg cell of the present embodiment is derived from a female of the genetic modification non-human organism described above.

In the present specification, the term "egg cell" means a female reproductive cell, including an oocyte and an unfertilized egg.

As the egg cell derived from the genetic modification non-human organism according to the present embodiment, in a case where the genetic modification non-human organism is a heterozygote having the Cas9 gene on one side of a homologous chromosome in the nuclear genome, there is an egg cell having, within the nucleus, the nuclear genome into which at least 3 copies of the Cas9 genes are introduced, and there is an egg cell not having the nuclear genome into which the Cas9 genes are introduced. Any of the egg cells can be utilized to modify a target gene. This is because protein or mRNA of maternally derived Cas9 (hereinafter will be referred to as maCas9) accumulates in the egg cell during the oogenesis and gradually decomposes after functioning to the 2-cell stage from the fertilized egg. Therefore, in a case where it is desired to produce the genetic modification non-human organism that does not have a foreign gene and in which a target gene is modified, it is preferable to use the egg cell not having, within the nucleus, the nuclear genome into which the Cas9 gene is introduced.

It is sufficient that the genetic modification non-human organism from which the egg cell is derived in the present embodiment have the nuclear genome into which at least 3 copies of the Cas9 genes are introduced, and protein or mRNA of maCas9 can accumulate in an oocyte during the oogenesis. In addition, the larger the copy number of the introduced Cas9 gene becomes, the more the accumulated protein or mRNA of maCas9 increases, which is preferable. The copy number of the introduced Cas9 gene may be 3, 4, 5, 6, 7, 8, 9, 10, 20, 50 or more, for example.

### <Fertilized Egg>

It is also hereby described fertilized eggs but they are not part of the invention. A fertilized egg obtained by fertilizing the egg cells derived from the genetic modification non-human organism having the nuclear genome into which at least 3 copies of the genes that code for Cas9 are introduced, and sperm derived from the same species of the organism.

A plurality of different genes or a plurality of locations in the same gene can be edited at the same time with high efficiency. It is possible to rapidly produce the genetic modification non-human organism at a low cost.

The fertilized egg can be obtained by artificially fertilizing the above-described egg cell derived from a female of the genetic modification non-human organism and sperm derived from the same species of the organism.

As the egg cell derived from the genetic modification non-human organism, in a case where the genetic modification non-human organism is a heterozygote having the Cas9 gene on one side of a homologous chromosome in the nuclear genome, there is an egg cell having, within the nucleus, the nuclear genome into which at least 1 copy of the Cas9 gene is introduced, and there is an egg cell not having the nuclear genome into which the Cas9 gene is introduced. Any of the egg cells can be utilized to modify a target gene. This is because protein or mRNA of maCas9 accumulates in the egg cell during the oogenesis and gradually decomposes after functioning to the 2-cell stage from the fertilized egg. Therefore, in a case where it is desired to produce the genetic modification non-human organism that does not have a foreign gene and in which a target gene is modified, it is preferable to use the egg cell not having, within the nucleus, the nuclear genome into which the Cas9 gene is introduced.

It is sufficient that the genetic modification non-human organism from which the egg cell is derived has the nuclear genome into which at least 3 copies of the Cas9 genes are introduced, and protein or mRNA of maCas9 can accumulate in an oocyte during the nuclear genome oogenesis. In addition, the larger the copy number of the introduced Cas9 gene becomes, the more the accumulated protein or mRNA of maCas9 increases, which is preferable. The copy number of the introduced Cas9 gene may be 3, 4, 5, 6, 7, 8, 9, 10, 20, 50 or more, for example.

Sperm may be sperm of the same species as the organism from which the egg cell was obtained and may be sperm derived from the genetic modification non-human organism having, within the nucleus, the nuclear genome into which at least one copy of the Cas9 gene is introduced, may be sperm derived from the genetic modification non-human organism not having the nuclear genome into which the Cas9 gene is introduced, or may be sperm derived from a wild-type organism. In a case where it is desired to prepare the genetic modification non-human organism that does not have the Cas9 gene and in which a target gene is modified, the sperm that does not have, within the nucleus, the nuclear genome into which the Cas9 gene is introduced or is derived from a wild-type organism is preferable.

In the egg cell not having, within the nucleus, the nuclear genome into which the Cas9 gene is introduced, protein or mRNA of maCas9 is accumulated, as described above. Therefore, in a case of a fertilized egg obtained by fertilizing the egg cell not having, within the nucleus, the nuclear genome into which the Cas9 gene is introduced, and the sperm that does not have, within the nucleus, the nuclear genome into which the Cas9 gene is introduced or is derived from a wild-type organism, protein or mRNA of maCas9 is transiently present excessively in a short period of the 2-cell stage from the fertilized egg. Accordingly, as shown in examples described later, mosaic mutations become significantly lower than those of a genomic editing method of the related art in which synthetic Cas9 mRNA and guide RNA are microinjected into the fertilized egg.

### <Method for Modifying Target Gene>

In one embodiment, the present invention provides a method for modifying a target gene including a step of introducing a guide RNA into the egg cell described above.

In one embodiment, the present invention further provides a method for modifying a target gene, including a step of introducing the guide RNA into the egg cell described above ; a step of cleaving the target gene at a cleavage site located upstream of a PAM (Proto-spacer Adjacent Motif) sequence by Cas9 expressed in the egg cell derived from the genetic modification non-human organism ; and a step of obtaining the modified target genes in a region determined by complementary binding of the guide RNA and the target gene, in which the target gene has the PAM sequence, and the guide RNA contains a polynucleotide including a base sequence complementary to an upstream base sequence of the PAM sequence in the target gene.

According to the method of the present embodiment, in vitro and in vivo editing of the target gene can be carried out simply and efficiently. In addition, the genetic modification non-human organism can be rapidly produced at a low cost.

In the present specification, the "target gene" is not particularly limited as long as the target gene is a gene having the PAM sequence, and examples thereof include a gene that can be studied as a gene related to lifestyle diseases such as cancer or heart disease.

In the present specification, the "PAM sequence" is a sequence recognizable by Cas9, and a length and a base sequence of the PAM sequence vary depending on the bacterial species from which Cas9 is derived. For example, S. pyogenes recognizes three bases of "NGG" (N represents any base).

Streptococcus thermophilus (S. thermophilus) has two Cas9s and recognizes 5 and 6 bases of "NGGNG" or "NNAGAA" (N represents any base) as a PAM sequence, respectively. It is preferable that the PAM sequence be "NGG", because the PAM sequence to be recognized is short and the editable target gene is unlikely to be limited.

In the present embodiment, it is sufficient that Cas9 expressed in the egg cell derived from the genetic modification non-human organism recognize the PAM sequence, "NGG", and examples thereof include Cas9 derived from S. pyogenes, and the like. In addition, as described in the section of "Genetic Modification Non-Human Organism" described above, Cas9 may be modified, or may be one obtained by expressing a Cas9 gene including a base sequence in which a codon is optimized.

In the present specification, the "guide RNA" is one that mimics a hairpin structure of a tracrRNA-crRNA chimera obtained by fusing a small RNA fragment (CRISPR-RNA: crRNA) containing a foreign sequence (guide sequence) and constituting the adaptive immune system that provides acquisition resistance against invading foreign nucleic acid in bacteria and archaebacteria, and RNA that is partially complementary to the crRNA (trans-activating crRNA: tracrRNA).

The guide RNA used in the present embodiment may be a tracrRNA-crRNA chimera synthesized in a state where crRNA containing the guide sequence and tracrRNA are fused or may be a tracrRNA-crRNA chimera in which crRNA containing the guide sequence and tracrRNA are separately produced and annealed before introduction.

The guide RNA in the present embodiment contains, at the 5' end region, a polynucleotide composed of a base sequence complementary to a base sequence from 1 base upstream of the PAM sequence in the target gene, to preferably 20 bases to 24 bases, and to more preferably 22 bases to 24 bases. Furthermore, the guide RNA may contain one or more polynucleotides which have a base sequence non-complementary to the target gene and is composed of a base sequence that is arranged in a symmetrical and complementary manner with one point as an axis and that can have a hairpin structure.

The method of the present embodiment will be described in detail below.

### [Introduction Step]

First, guide RNAs are introduced into egg cells described above. As a method for introducing the guide RNAs, known gene introduction methods (for example, calcium phosphate method, electroporation method, lipofection method, coagulation method, microinjection method, particle gun method, DEAE-dextran method, and the like) can be used. Among these, the electroporation method is preferable because the operation is simple. In the genetic modification method utilizing the CRISPR system of the related art, the number of Cas9 mRNA was large and an introduction efficiency was poor. However, in the method of the present embodiment, egg cells derived from a genetically modified non-human mammal selected from the group consisting of a mouse, rat, hamster, guinea pig, rabbit, dog, cat, horse, cow, sheep, pig, goat, marmoset, and monkey, are used, and therefore it is possible to easily and efficiently introduce the guide RNAs by the electroporation method and the like.

In a case where two or more locations of one target gene are modified, or in a case where two or more of genes are used as target genes, two or more types of the guide RNAs containing, in the 5' end region, a polynucleotide including a base sequence complementary to the base sequence of each gene, may be designed so as to be introduced. Accordingly, it is possible to edit a plurality of different genes or a plurality of locations in the same gene at the same time with high efficiency.

In the method of the present embodiment, a foreign gene may be introduced together with the guide RNA. By introducing a foreign gene together, it is possible to obtain the genetic modification non-human organism having the nuclear genome in which after cleavage of a target gene, the foreign gene is inserted into the target gene by homologous recombination.

In addition, it is preferable that the foreign gene not have a PAM sequence. In the foreign gene, for example, in a case where the PAM sequence is changed to a non-PAM sequence by one base substitution not changing the amino acid sequence, and the like, it is possible to insert the foreign gene on the nuclear genome by homologous recombination and it is also possible to prevent the foreign gene from being cleaved again by the endogenous Cas9.

It is preferable that the foreign gene be introduced using a vector containing the foreign gene. In the vector containing the foreign gene, it is preferable that a DNA having a sequence homologous to a site into which a target gene is insert be linked to the 5' end and the 3' end of the foreign gene. The number of bases of the DNA having a sequence homologous to a site into which a target gene is inserted is preferably 100 bases to 300 bases. In the vector containing the foreign gene, the promoter, the polyadenylation signal, the NLS, the fluorescent protein marker gene, which are described in the above section, "Genetic Modification Non-Human Organism", and the like may be operably linked to the 5' end or the 3' end of the foreign gene.

In a case of use in the present specification, the "vector" is a tool that enables or facilitates the transfer of entities from one environment to another environment. For example, some vectors used in recombinant DNA technology enable entities such as segments of DNA (for example, heterologous DNA segments such as heterologous cDNA segments) to be transferred into non-human organism cells. In the present embodiment, the vector includes viral vectors (for example, lentiviral vectors, baculovirus vectors, adenovirus/adeno-associated viral vectors, and the like), bacterial vectors, protozoal vectors, DNA vectors, or recombinant vectors which may contain recombination thereof.

### [Cleavage Step]

Cas9 expressed in the egg cell derived from the genetic modification non-human organism and the guide RNA are combined in mild conditions in vitro or in vivo so as to form a Cas 9-guide RNA complex. The mild conditions indicate a temperature and a pH at which a protein does not decompose or denature, and the temperature may be 4°C to and 40°C, and the pH may be 4 to 10. Accordingly, the Cas9-RNA complex can be formed even in the body of the living genetic modification non-human organism.

In the Cas9-RNA complex, some of the guide RNA binds to the target gene and Cas9 recognizes the PAM sequence in the target gene. Subsequently, the target gene is cleaved at a cleavage site located 3 bases upstream so as to produce blunt ends. More specifically, Cas9 recognizes the PAM sequence, a double helix structure of the target gene is peeled from the PAM sequence as a starting point, followed by annealing with the base sequence complementary to the target gene in the guide RNA, and therefore the double helix structure of the target gene is partially loosened. At this time, Cas9 cleaves the phosphodiester bond of the target gene at the cleavage site located 3 bases upstream of the PAM sequence so as to create blunt ends.

### [Modification Step]

Subsequently, in a region determined by complementary binding between the guide RNA and the target gene, it is possible to obtain a target gene subjected to the modification according to the purpose.

In the present specification, the term "modification" means that the base sequence of the target gene is changed. Examples of thereof include a change in the base sequence of a target double-stranded polynucleotide by cleavage of a target gene and insertion of an exogenous sequence after the cleavage (physical insertion or insertion by replication via homology directed repair), a change in the base sequence of a target gene by non-homologous end joining (NHEJ: DNA ends generated by cleavage are bonded again) after the cleavage, and the like. By the modification of the target gene in the present embodiment, introduction of mutation into the target gene or destruction of a function of the target gene is possible. Therefore, in the present embodiment, in a case of using the fertilized egg, a genetic modification non-human organism in which a function of the target gene is destroyed (knockout) or substituted (knockin) can be easily produced.

It is also hereby described a method for modifying a target gene including a step of introducing a guide RNA into the cell derived from the genetic modification non-human organism described above, or the fertilized egg described above, but this method is not part of the invention.

It is further described a method for modifying a target gene, including a step of introducing the guide RNA into the cell derived from the genetic modification non-human organism described above or the fertilized egg described above; a step of cleaving the target gene at a cleavage site located upstream of a PAM (Proto-spacer Adjacent Motif) sequence by Cas9 expressed in the cell derived from the genetic modification non-human organism or the fertilized egg derived from the genetic modification non-human organism; and a step of obtaining the modified target genes in a region determined by complementary binding of the guide RNA and the target gene, in which the target gene has the PAM sequence, and the guide RNA contains a polynucleotide including a base sequence complementary to an upstream base sequence of the PAM sequence in the target gene.

It is sufficient that Cas9 expressed in the cell derived from the genetic modification non-human organism or the fertilized egg derived from the genetic modification non-human organism recognize the PAM sequence, "NGG", and examples thereof include Cas9 derived from S. pyogenes, and the like. In addition, as described in the section of "Genetic Modification Non-Human Organism" described above, Cas9 may be modified, or may be one obtained by expressing a Cas9 gene including a base sequence in which a codon is optimized.

### [Introduction Step]

First, guide RNAs are introduced into cells derived from the genetic modification non-human organism or the fertilized eggs described above. As a method for introducing the guide RNAs, known gene introduction methods (for example, calcium phosphate method, electroporation method, lipofection method, coagulation method, microinjection method, particle gun method, DEAE-dextran method, and the like) can be used. Among these, the electroporation method is preferable because the operation is simple. In the genetic modification method utilizing the CRISPR system of the related art, the number of Cas9 mRNA was large and an introduction efficiency was poor. However, in the method of the present embodiment, the cells derived from genetic modification non-human organism in which Cas9 can be expressed, or fertilized eggs are used, and therefore it is possible to easily and efficiently introduce the guide RNAs by the electroporation method and the like.

In a case where an introduction target is a cell derived from the genetic modification non-human organism, the guide RNA can be introduced into a living genetic modification non-human organism. In this case, the introduction method may be a method known to those skilled in the art, such as intra-arterial injection, intravenous injection, subcutaneous injection, intranasal introduction, transbronchial introduction, intramuscular introduction, percutaneous introduction, or oral introduction, and the intravenous injection is preferable.

In addition, in a case where a tissue-specific promoter is operably linked to the upstream (5' side) of the Cas9 gene, by the guide RNA being directly introduced into the entire body of the genetic modification non-human organism or target tissues, the genetic modification is performed only in the target tissues by the guide RNA and Cas9, and therefore the dynamics of diseases can be observed.

In a case where the introduction target is a fertilized egg fertilized by the above egg cell, or a fertilized egg, it is preferable to use a fertilized egg of 4 hours to 24 hours, preferably 6 hours to 12 hours, and more preferably 6 hours to 8 hours after fertilization, in which male pronucleus is developed. With the time after fertilization being within the above range, an expression level of protein or mRNA of maCas9 becomes high, and an embryo of a so-called mosaic-type in which both the cell in which the target gene is modified and the cell in which the target gene is not modified are present, is prevented from being generated. Therefore, it is possible to modify the target gene with high efficiency.

In addition, as described in the above section, "Fertilized Eggs", in a case where it is desired to produce the genetic modification non-human organism which does not have, within in the nucleus, the nuclear genome into which the Cas9 gene is introduced, and in which the target gene is modified, a fertilized egg to be used is preferably a fertilized egg obtained by fertilizing an egg cell not having, within the nucleus, the nuclear genome into which the Cas9 gene is introduced, and sperm derived from an organism not having, within the nucleus, the nuclear genome into which the Cas9 gene is introduced, or a wild-type organism. In the egg cell not having, within the nucleus, the nuclear genome into which the Cas9 gene is introduced, protein or mRNA of maCas9 is accumulated. Therefore, in a case where the fertilized egg to be used is a fertilized egg obtained by fertilizing an egg cell not having, within the nucleus, the nuclear genome into which the Cas9 gene is introduced, and sperm derived from an organism not having, within the nucleus, the nuclear genome into which the Cas9 gene is introduced, or a wild-type organism, protein or mRNA of maCas9 is transiently present excessively in a short period of the 2-cell stage from the fertilized egg. Accordingly, as shown in the examples described later, mosaic mutations become significantly lower than those of the genomic editing method of the related art in which synthetic Cas9 mRNA and guide RNA are microinjected into the fertilized egg.

In a case of introducing the guide RNA into the fertilized egg, a concentration of the guide RNA is not particularly limited, but as the concentration becomes higher, genetic modification can be performed with higher probability. A concentration of the guide RNA may be, for example, 1 ng/µL to 300 ng/µL.

In addition, in a case where the fertilized egg is derived from the genetic modification non-human animal (particularly, a non-human mammal), after introduction of the guide RNA, the fertilized egg is implanted to the uterus or the oviduct of the corresponding non-human animal so as to be generated, and therefore it is possible to easily obtain the genetic modification non-human animal.

### <Application and Utilization Method>

The present disclosure also relates to the following aspects which are not part of the invention.
It is described a composition and a method for performing genetic modification. In contrast to previously known methods for targeted genetic recombination, this enables efficient and inexpensive implementation and thus is adaptable to any cell or non-human organism Any segment of the target gene of a cell or a non-human organism can be modified by this method. This method utilizes both a homologous recombination process in which all cells are endogenous and a non-homologous recombination process.

It is also described a method for performing targeted DNA insertion or targeted DNA deletion. This method includes a step of transforming a cell using a nucleic acid construct having a donor DNA. The scheme concerning the DNA insertion or the DNA deletion after cleavage of a target gene can be determined by those skilled in the art according to known methods.

In particular it is utilized in both somatic cells and reproductive cells and provides genetic manipulation at a specific locus.

It is also described a method for breaking genes in somatic cells. The gene overexpresses products harmful to cells or organisms and expresses products harmful to cells or organisms. Such a gene can be overexpressed in one or more cell types that arise in diseases. Disruption of the overexpressed gene according to the method of the present invention may lead an individual suffering from a disease caused by the overexpressed gene to be healthier.

That is, disruption of only a small proportion of the genes of the cells works and an expression level thereof is reduced, obtaining a therapeutic effect.

It is also described a method for breaking a gene in reproductive cells. A cell in which a specific gene is disrupted can be selected so as to produce a non-human organism not having a function of the specific gene. In the cell in which the gene is disrupted, the gene can be completely knocked out. A deficiency in a function of this specific cell may have a therapeutic effect.

It is further provided insertion of a donor DNA that codes for a gene product. This gene product has a therapeutic effect in a case of constitutive expression.

For example, in a population of pancreatic cells, in order to cause the insertion of a donor DNA that codes for an active promoter and an insulin gene, there is a method in which the donor DNA is inserted into an individual suffering from diabetes. The population of pancreatic cells containing exogenous DNA can then produce insulin and thus can treat the individual suffering from diabetes.

In addition, it is possible to select cells that produce cells which are utilized in reproductive cells and in which insertion is performed in a designed manner and all subsequent cell divisions have designed genetic modification.

This described method can be applied to non-human organisms, cultured cells, cultured tissues, and cultured nuclei (including cells, tissues, or nuclei that can be used to regenerate intact organisms), and non-human gametes (for example, eggs or sperm at various stages of their development).

In addition, it can be applied to cells derived from any organism (including, but are not limited to, insects, fungi, rodents, cows, sheep, goats, chickens, and other agronomically important animals, and other mammals (including, but are not limited to, dogs, cats, and humans).

In addition, it can be applied in the production of non-human animal disease models (for example pathological model mice).

### Examples

Hereinafter, the present invention will be described in further detail with reference to examples and the like, but the present invention is not limited to these examples and the like.

### [Experimental Material]

Base sequences of guide RNAs and primers used in the present example are shown in Tables 1 to 3. In Table 1, bold letters (three bases on the 3' side of a base sequence) indicate base sequences homologous to a PAM sequence on a target gene.

**[Table 1]**

| Guide RNA | | | | |
|---|---|---|---|---|
| ID | Target Sequence (5' → 3') | Location | Gene Bark# | SEQ ID NO |
| Alb-gRNA | CGCAGATGACAGGGTAAGGA**AGG** | Near Alb exon 7 | NM009654 | 18 |
| Amy-gRNA | ATATGTATTCGATCCCACGT**TGG** | Amv exon 2 | NM010491 | 19 |
| Crlr-gRNA | ATGTGATCCGTTGGCAACTT**AGG** | Crlr exon 6 | NM018782 | 20 |
| Et-1-gRNA | GCGCGTCGTACCGTATGGAC**TGG** | Et-1 exon 3 | NM010104 | 21 |
| Ggat1-gRNA | GAGAAAATAATGAATGTCAA**AGG** | Ggta1 exon 4 | NM001145821 | 22 |
| Hprt-gRNA | GGGACTGCGGGTCGGCATGA**CGG** | Hprt exon 1 | NM013556 | 23 |
| Imdn-gRNA | GGCTGCCAGACAACGTGCCG**GGG** | Imdn exon 2 | NM182928 | 24 |
| Klf5-gRNA | GGTCAGCACCCGCGTGGGCA**TGG** | Klf5 exon 1 | NM009769 | 25 |
| R1-gRNA | GCTCTGCTTGCCATGGCCCC**GGG** | Ramp1 exon 1 | NM001168392 | 26 |
| R2-gRNA | GGTGAGCACCGTGTCGGGCC**CGG** | Near Ramp2 exon 1 | NM019444 | 27 |

**[Table 2]**

| Primer 1 | | |
|---|---|---|
| ID | Sequence (5' → 3') | SEQ ID NO |
| Amv-IS | CGGTGAAGGGTGTTGTGTGA | 28 |
| Amy-1A | ACACAGTCATCAAGCACAAGC | 29 |
| Alb-7S | CTAACGCCTGCTGGTGGTCACAGT | 30 |
| Alb-7A | TGTCTAGCCCCTGCTTCTCT | 31 |
| CAG1620-S | GCTCTAGAGCCTCTGCTAACCATGT | 32 |
| CAGGS-A | GCGGAACTCCATATATGGGCTATGAACTAATG | 33 |
| Cas9 215-A | CGCCGTGCTGTTCTTTTGAGC | 34 |
| Cas9ATG-S | GCCACCATGGACAAGAAGTACTCCATTG | 35 |
| Cas9Cla-A | CCATCGATTCACACCTTCCTCTTCTTCT | 36 |
| Cas9E-S | AAGCGAATTCTCCAAAAGAGTGAT | 37 |
| Crlr-5S | AGAGTTTGCTGAGGCAGGTC | 38 |
| Crlr-5A | CCAGCCCATGTCTCTTCCTA | 39 |
| Et-1-1S | TGGTGTGGCTGTAGTCCTTC | 40 |
| Et-1-1A | CAAGTCAAAGGGGCCTCCAC | 41 |
| Ggta1-S | TCAACCACACAGCTGTTTCTC | 42 |
| Geta1-A | CTGGCACCAGTCACAGGGAATG | 43 |
| Gtpbp10-S | GTTGTTATAATCTTCTAACAG | 44 |
| Gtpbp10-A | CTGGGTGGAACAATTGATCGT | 45 |
| Hprt-1S | TGAGCCATTGCTGAGGCG | 46 |
| Hprt-1A | CGCGCCTGATCCTTCCTG | 47 |
| Hprt-SF | TGTTGGATACAGGCCAGACTTTGTT | 48 |
| Hprt-SR | TCTTAGGCTTTGTATTTGGCTTTTC | 49 |
| Imdn-1S | ACGCAGGTACCAACCAATCT | 50 |
| Imdn-1A | CCCGGGTTCTTCCCTTATGT | 51 |
| Klf5-1S | GGTACGCGCTCTCTTAGGTT | 52 |
| Klf5-1A | GCGTGTTTCAGATCGTCTCC | 53 |

**[Table 3]**

| Primer 2 | | |
|---|---|---|
| ID | Sequence (5' → 3') | SEQ ID NO |
| Ramp1-2S | GTCAGAGCGAGGTGCTGAGT | 54 |
| Ramp1-2A | CCCATCACCACTCTACTGTCTTG | 55 |
| Ramp1-SF | GCACTGGTGGTCTGGAGGA | 56 |
| Ramp1-SR | CCCTCATCACCTGGGATACCT | 57 |
| Ramp2-SF | TGTGCCTCCCTCCGCTGTT | 58 |
| Ramp2-SR | TTACTCCTCCACACCACAAGA | 59 |
| Ramp2-5S | CCGAGCTGGAAGCGAGAG | 60 |
| Ramp2-6A | GACCTCTCCGTCCGGTCT | 61 |
| Ramp3-S | TCGAATTCATCTTAGTTGGCCATGAAGAC | 62 |
| Ramp3-A | TCGAATTCACAGCAGCCGATCAGTGTGCTTG | 63 |
| Sp#2 | GTGGCTGAATGAGACTGGTGTCGAC | 64 |
| Sp-Bottom | CGAAGAGTAACCGTTGCTAGGAGAGACCGTGGCTGAATGAGACTGGTGTCGACACTAGTGG | 65 |
| Sp-EV Top | CCACTAGTGTCGACACCAGTCTCTAATTTTTTTTTCAAAAAA | 66 |
| NFL (D10A) S | CTGCAGGAATTCGCCACCATGGGACCTAAG | 81 |
| NFL (D10A) A | GCTGTTTGTGCCGATAGCCAGTCCAATAGAG | 82 |

**[Table 4]**

| Off-target analytical primer | | | | |
|---|---|---|---|---|
| Gene | | ID | Sequence (5' → 3') | SEQ ID NO |
| Ramp1 | Ccdc3 (NM_028804) | OfftR1-11 | TATATCTGTTTTCCAACCCCAGCTC | 67 |
| | | OfftR1-12 | TGGGGTACACGTAGACCTTTTATGA | 68 |
| | Trappc13 (NM_001093760) | OfftR1-21 | TCTCTGGCTGAATTGTTTTACACAGG | 69 |
| | | OfftR1-22 | AAAGCAGTCAGCGAACCTTTTTG | 70 |
| | Krt5 (NM_027011) | OfftR1-31 | ATGTCTCGCCAGTCCAGTGTGTC | 71 |
| | | OfftR1-32 | CACCACCAAAACCAAATCCACTG | 72 |
| Ramp2 | Chpf (NM_001001565) | OfftR2-11 | ACTCAGACGTCTTCGCACCTGTC | 73 |
| | | OfftR2-12 | CGCCACATAGTCGGAGTTGTAGA | 74 |
| | B3gat1 (NM_029792) | OfftR2-21 | CACCTCTGCAGATGAGGGAAGTG | 75 |
| | | OfftR2-22 | GGGCATCACCTCGCAGCTTATAG | 76 |
| | Sema6b (NM_001130456) | OffLR2-31 | CGCCTTTGACATGAACCAAGTG | 77 |
| | | OfftR2-32 | ACTCACTGTAGCCTCGGGACCTC | 78 |

Furthermore, the guide RNA was prepared by the following method.

First, using a pgRNA-GFP-T1 vector (#41819, manufactured by Addgene) as a template, a guide RNA to which a TTTTTT site was added at the 5' end was amplified by PCR. Subsequently, the guide RNA was inserted into a pBluescript II vector (manufactured by Agilent Technologies), and therefore a pBS-T7-gRNA plasmid was obtained. A base sequence of the guide RNA on the obtained plasmid was confirmed by sequencing.

Subsequently, by using MEGAshortscript (registered trademark) T7 kit (manufactured by Life Technologies), the guided RNA was prepared from the plasmid linearized with EcoRI.

### [Example 1] Establishment of Cas9-Highly Expressing Mouse Line

### (1) Design of Construct for Inserting Cas9 Gene

Using, as a template, phCas9 (manufactured by Addgene) containing a Cas9 gene (base sequence: SEQ ID NO: 17) in which a codon was optimized by gene synthesis, PCR was carried out using a Cas9ATG-S primer and a Cas9Cla-A, and therefore an amplified gene product was obtained. Subsequently, by PCR, a SV40 nuclear localization signal (NLS) and a base sequence of a Flag tag, or the base sequence of only the Flag tag was inserted immediately after a start codon (ATG) of a Cas9 gene. Therefore, NFCas9 (Cas9 gene having the NLS and the Flag tag) and FCas9 (Cas9 gene having only the Flag tag) were obtained.

Subsequently, each of NFCas9 and FCas9 was inserted into the EcoRI site of a pCAGGS vector so as to construct pCAG-NFCas9pA and pCAG-FCas9pA, respectively.

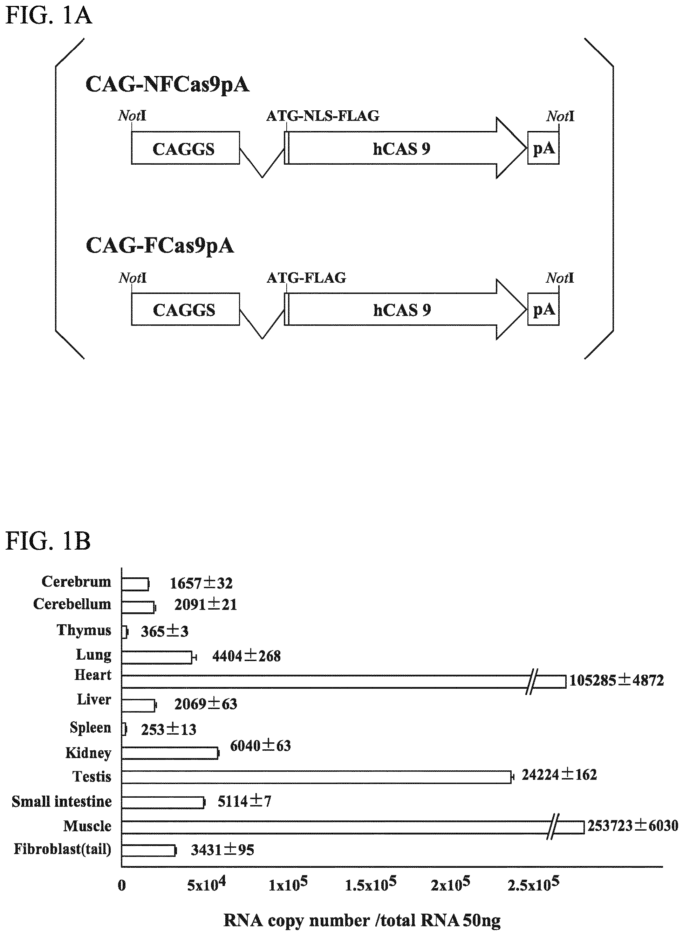

These were each linearized with NotI, and therefore CAG-NFCas9pA and CAG-FCas9pA sites were purified (refer to FIG. 1A).

### (2) Injection into Fertilized Mouse Egg

In the present example, mice of BDF1 strain, C57BL6/J-Jcl strain (hereinafter will be referred to as "B6"), and ICR strain were purchased from Crea Japan Company Limited and used.

A fertilized egg to be used was produced by fertilizing sperm of a male B6 mouse and an egg cell of a female BDF 1 mouse by using a general in vitro fertilization (IVF) method.

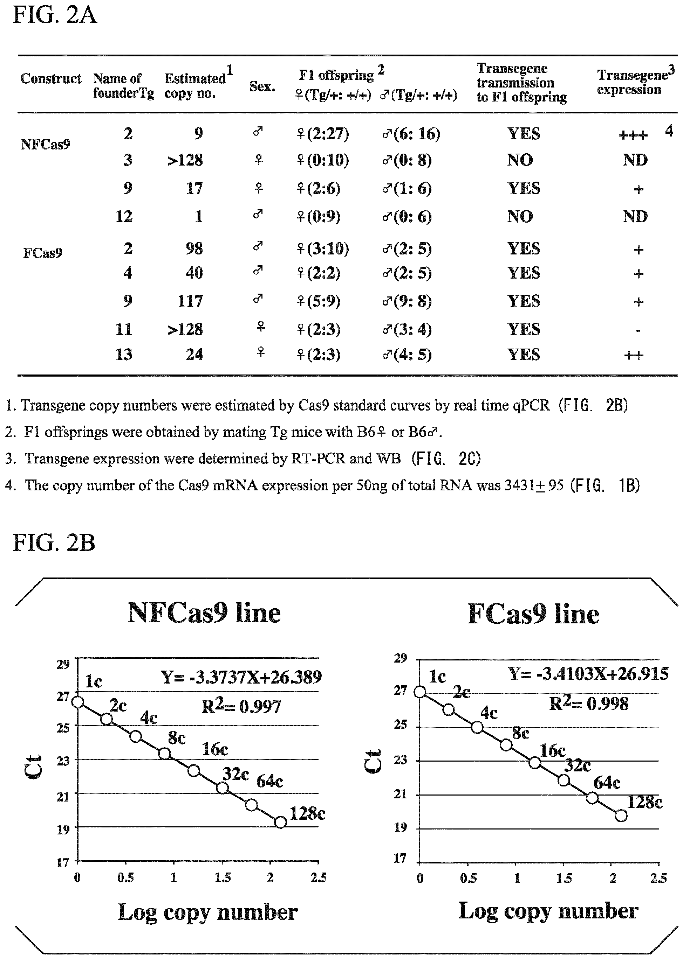

Subsequently, an injection solution was prepared so that each of the vectors containing the two types of Cas9 genes prepared in (1) became 2 ng/µL. Subsequently, the injection solution was injected into the pronuclei of the fertilized mouse eggs (56 for NFCas9 and 83 for FCas9). Subsequently, the fertilized egg into which the gene was introduced was implanted into the oviduct of an ICR pseudopregnant female mouse, and therefore F1 generation of the Cas9 transgenic mouse was obtained. Identification of introduction of Cas9 was carried out by PCR using a CAG1620-S primer and a CAS9215-A primer. As a result, 4 lines of a NFCas9 mouse and 5 lines of FCas9 were established. The characteristics of each line are shown in FIG. 2A.

Based on FIG. 2A, it was confirmed that the Cas9 transgene was transferred to the pup mouse in all 2 lines of the NFCas9 mouse and 5 lines of the FCas9 mouse.

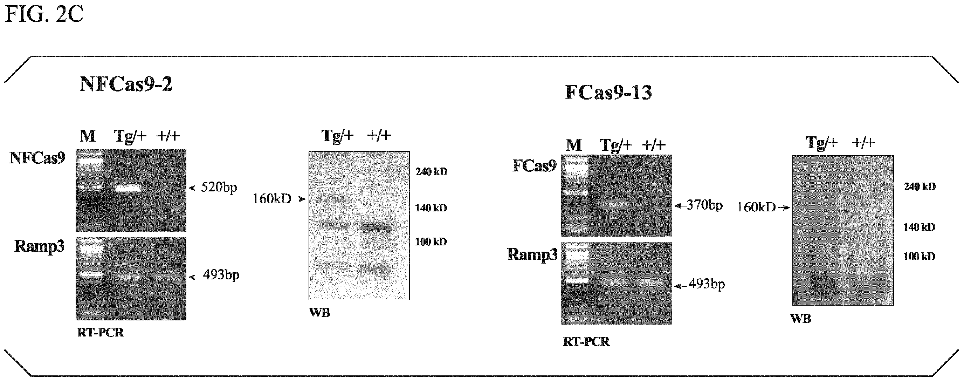

Expression of Cas9 in fibroblasts isolated from the F1 generation of all 7 lines was confirmed by quantitative real-time PCR, RT-PCR, and western blot analysis (refer to FIG. 2C).

A method for producing a calibration curve of Cas9 (refer to FIG. 2B) for quantitative real-time PCR is as follows.

PCR was carried out using a DNA solution containing, as a template, 100 ng of genomic DNA and a dilution series of a pCAG-NFCas9 vector or a pCAG-FCas9 vector (1, 2, 4, 8, 16, 32, 64, and 128-fold copies per 100 ng genomic DNA), and a calibration curve was drawn from an amount of the obtained PCR product.

Based on FIG. 2A, the NFCas9-2 line among the established mice highly expressed Cas9 and was thus used for future analysis.

### (3) Confirmation of Cas9-Introduced Locus in NFCas9-2 Line

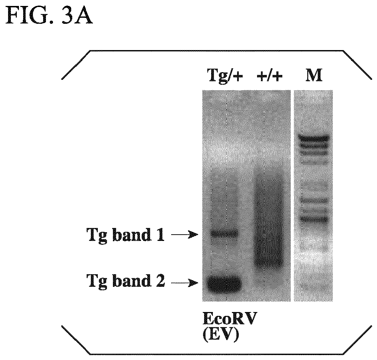

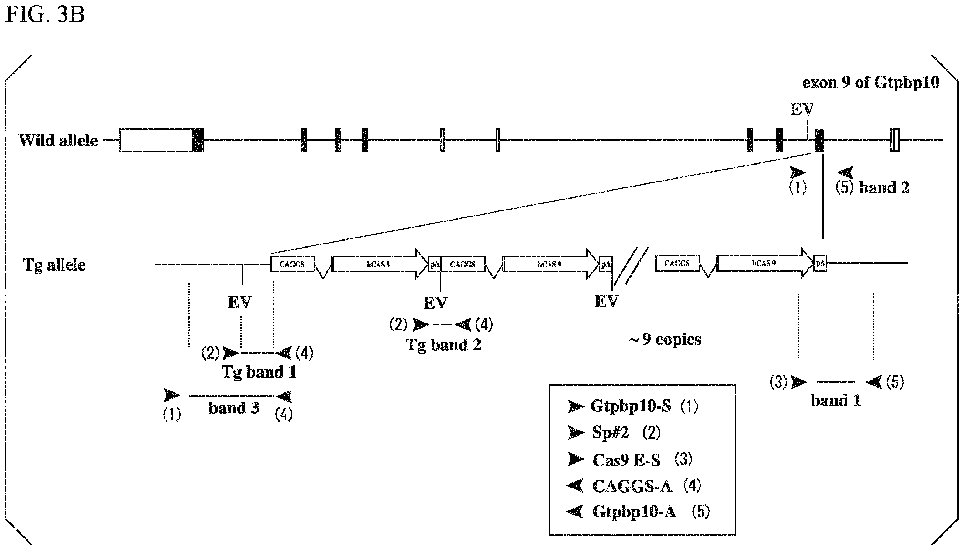

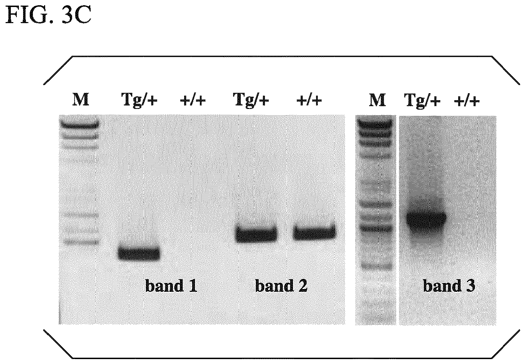

A locus into which Cas9 was introduced in the NFCas9-2 line was identified by being subjected to splinkerette PCR (spPCR) by using EcoRV splinkerette oligonucleotide (sp-EV Top primer and sp-Bottom primer) (refer to Potter, C. J. & Luo, L. Splinkerette PCR for mapping transposable elements in Drosophila. PloS One 5, e10168 (2010)). Furthermore, in order to ensure the result of spPCR, a PCR product of the genomic DNA of the NFCas9-2 line was obtained using a Gtpbp10-S primer, an Sp#2 primer, a Cas9E-S primer, a CAGGS-A primer, and a Gtpbp10-A primer. The results are shown in FIGS. 3A to 3C.

Based on FIGS. 3A to 3C, it was found that the NFCas9-2 line had nine NFCas9 transgenes in the cells, which were inserted into the Gtpbp10 gene.

### [Test Example 1] Confirmation Test on Genetic, Morphological, and Physiological Properties of NFCas9-2 Line Mouse

### (1) Crossing Using NFCas9-2 Line Mouse

Male or female mice of the NFCas9-2 line were crossed with the NFCas9-2 line mouse and a wild-type BDF1 strain or a wild-type B6 strain mouse. As a result, although detailed data are not shown, a transmission rate of the transgene to the pup was in accordance with Mendel's law.

In addition, the NFCas9-2 line also grew normally in an individual into which NFCas9 was homozygously introduced, and an average number of pups was equivalent to that of a wild-type mouse of the BDF1 strain (6 pups/birth).

### (2) Confirmation of Constant Expression Level of Cas9 mRNA

Cas9 mRNA in each tissue isolated from the NFCas9-2 line mouse was confirmed by quantitative real-time PCR. The results are shown in FIG. 1B.

Based on FIG. 1B, it became clear that expression of Cas9 mRNA was particularly high in the heart, muscle and testis.

### (3) Confirmation of Morphological Change in NFCas9-2 Line Mouse

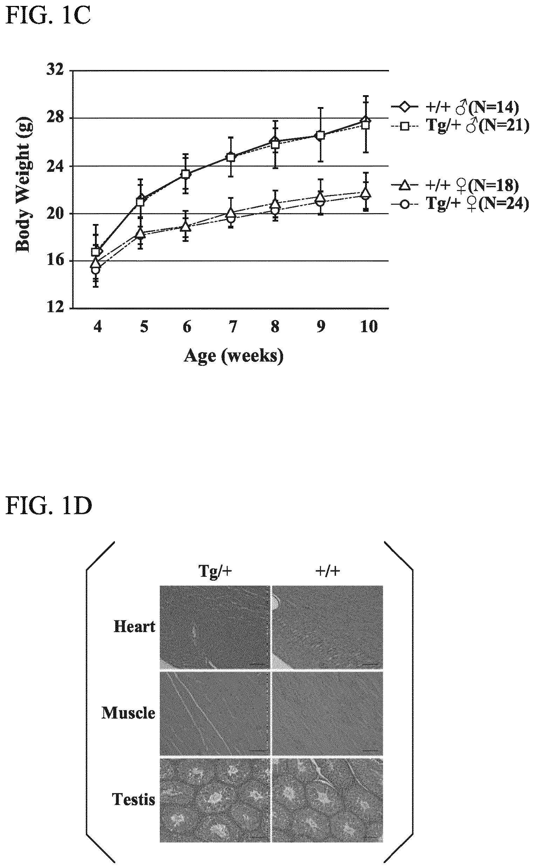

Crossing of a female NFCas9-2 line mouse of with a wild-type BDF1 strain mouse was carried out. Subsequently, weights of the obtained littermates, a mouse of heterozygous NFCas9 (hereinafter will be referred to as "Tg/+ mouse") and a mouse not having NFCas9 (hereinafter will be referred to as "+/+ mouse"), were each measured from 4 weeks old to 10 weeks old. The results are shown in FIG. 1C.

Based on FIG. 1C, in the Tg/+ mouse and +/+ mouse, an increase in weight tended to be the same and no difference in morphological change was recognized.

In addition, in the Tg/+ mouse and +/+ mouse, each tissue was taken out and a tissue fragment was produced. Subsequently, hematoxylin and eosin stain was carried out by a general method. The results are shown in FIG. 1D.

Based on FIG. 1D, in the heart, muscle, and testis, high expression of Cas9 mRNA was recognized in the Tg/+ mouse, and no morphological difference between +/+ mouse was recognized.

### (4) Confirmation of Physiological Marker of NFCas9-2 Line Mouse

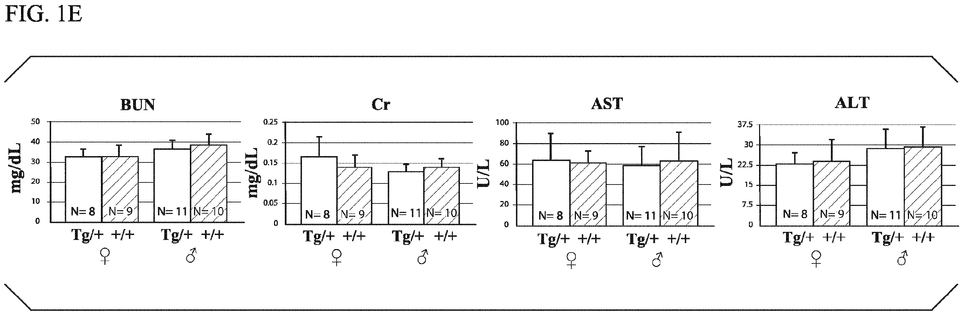

Blood was collected from 10-week-old Tg/+ mouse and +/+ mouse, and blood urea nitrogen (BUN), creatinine (Cr), aspartate aminotransferase (AST), and alanine aminotransferase (ALT), which are generally known as physiological markers in serum, were tested in SRL Corporation. The results are shown in FIG. 1E.

Various physiological markers were confirmed based on FIG. 1E.

### [Test Example 2] Ramp2 Genetic Modification Test

In the fertilized eggs derived from the Cas9 mouse, protein and mRNA of Cas9 (both or any one thereof) (hereinafter will be referred to as maCas9) were accumulated in the cell as a maternal factor, and it was considered that the Cas9 transgene is not required for genomic editing, and therefore the following test was carried out.

### (1) Introduction of Guide RNA

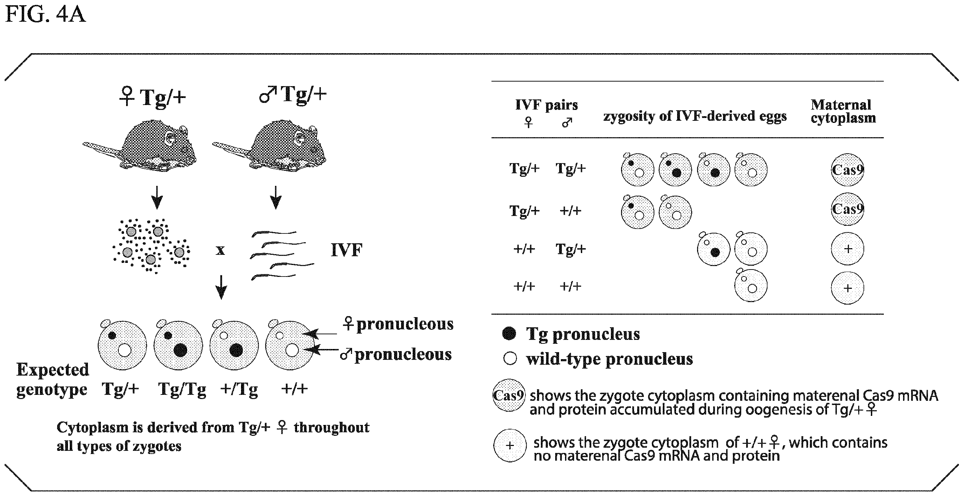

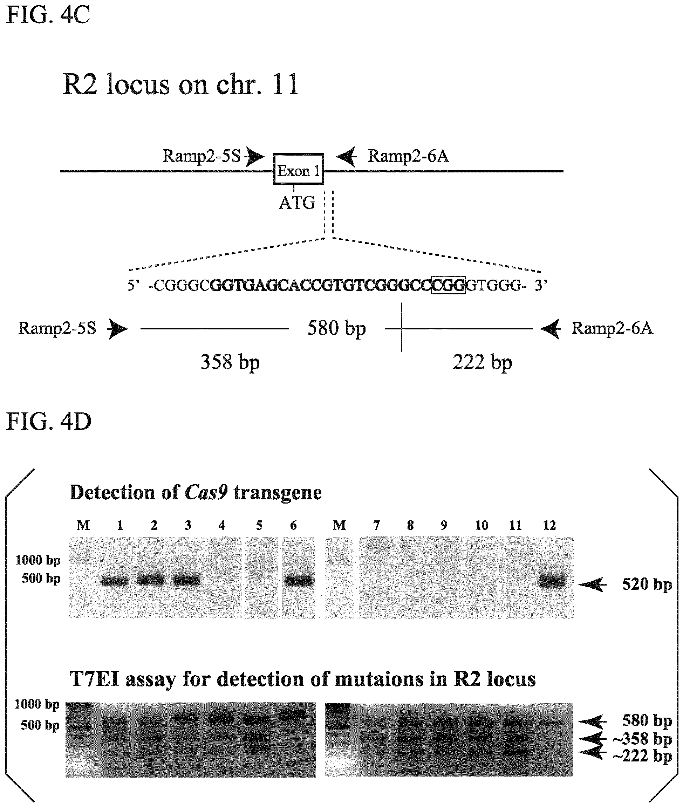

Only R2-gRNA (SEQ ID NO: 27) for Ramp2 gene modification was microinjected into the pronucleus of the fertilized eggs obtained by artificial fertilization of various crossing pairs. A genotype in the fertilized eggs produced by crossing is shown in FIG. 4A. In addition, a base sequence homologous to the Ramp2 gene in R2-gRNA is shown in FIG. 4C. In FIG. 4A, subsequently, the injected fertilized eggs were cultured to blastocysts in potassium-supplements simplex-optimized medium (KSOM).

### (2) Determination of Cas9 Genotype

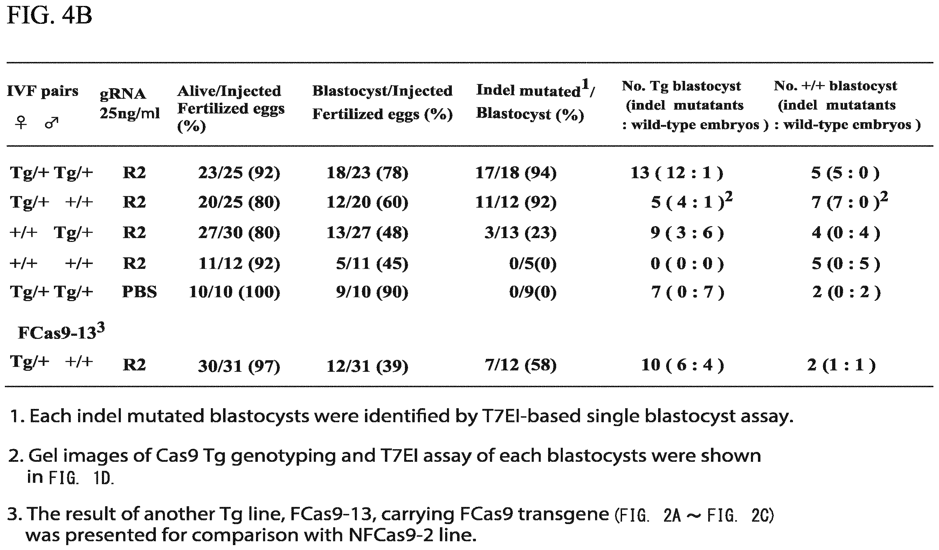

In regard to one embryo of the blastocysts, Cas9 genotype was determined using the same method as in (3) of Example 1. The results are shown in FIG. 4B.

### (3) T7 endonuclease I (T7EI) Assay

PCR was carried out from a nuclear extract of one embryo of the blastocysts using a Ramp 2-5S primer and a Ramp 2-6A, and therefore a PCR product was obtained. The PCR product was added to T7 endonuclease I (manufactured by New England BioLabs Japan), and therefore a DNA fragment was obtained. Subsequently, a fragment cleaved by agarose electrophoresis was confirmed. The results are shown in FIG. 4D.

Based on FIGS. 4B and 4D, in a case where artificial fertilization was carried out between the Tg/+ mice, or between a Tg/+ female mouse and a +/+ male mouse, it was found that an indel mutation (a genetic mutation due to base insertion or deletion) occurred at a high rate of 92% to 94%. Accordingly, it became clear that genomic editing is possible at a high rate by Cas9 in the Tg fertilized egg.

On the other hand, in a case where artificial fertilization was carried out between a +/+ female mouse and a Tg/+ male mouse, an indel mutation was 23%, which is a low rate. Furthermore, the inventors of the present invention have investigated a relationship between a genotype of a blastocyst and a genomic editing ability, and therefore have found that an indel mutation occurs at a high rate even in a blastocyst in which the Cas9 transgene is not present.

In addition, based on FIG. 4B, in a case where in vitro fertilization was carried out between Tg/+ mice or between a Tg/+ female mouse and a +/+ male mouse, in all +/+ blastocysts, a Ramp 2 locus (5 + 7/5 + 7) had a mutation. It is known that genes on the genome are not yet expressed in cells of a fertilized egg stage. Transcripts of genes on the genome are gradually made until the 2-cell stage.

Therefore, based on this result, it is considered that protein and mRNA of maCas9 (both or any one thereof) are responsible for genomic editing in the fertilized egg.

In addition, as shown in Fig. 4B, it was possible to confirm this phenomenon in another line, FCas9-13.

### [Test Example 3] Ramp1 Gene and Ramp2 Gene Modification Test

### (1) Introduction of Guide RNA

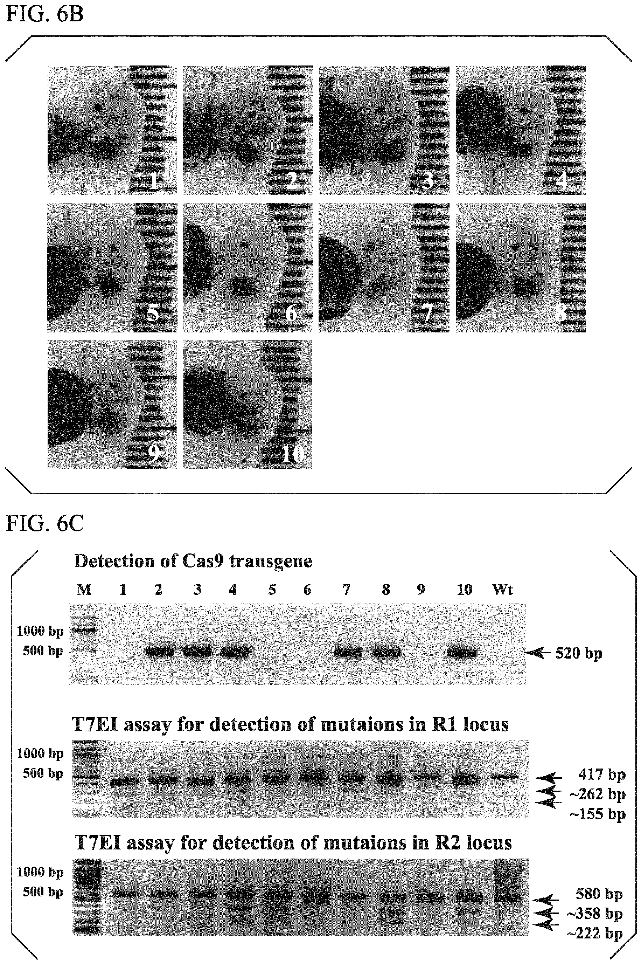

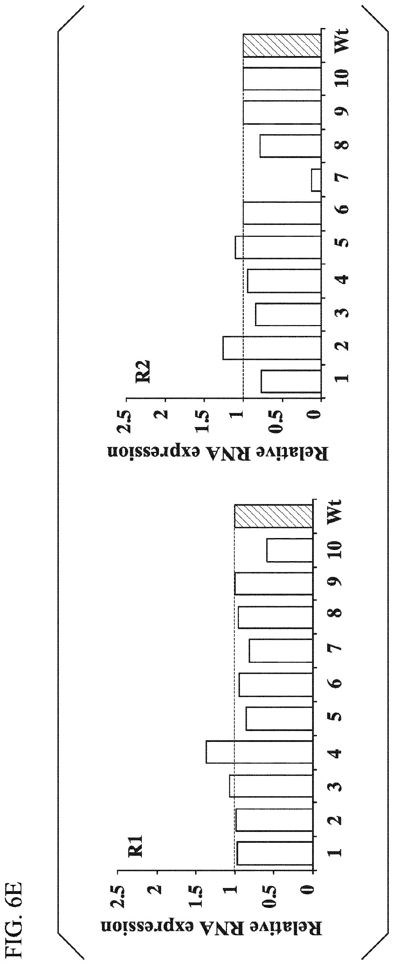

Any one of or both of R1-gRNA (SEQ ID NO: 26) for Ramp1 gene modification and R2-gRNA (SEQ ID NO: 27) for Ramp2 gene modification were microinjected into the pronucleus of the fertilized eggs obtained by artificial fertilization of a Tg/+ female mouse and a +/+ male mouse. Subsequently, the injected fertilized eggs were cultured to blastocysts in potassium-supplements simplex-optimized medium (KSOM). Some of the blastocysts into which R1-gRNA and R2-gRNA were introduced at the same time were implanted into the uterus of an ICR strain pseudopregnant female mouse. 13.5 to 15.5 days after implantation, embryos were obtained. The form of embryos of 10 pups 13.5 days after the implantation is shown in FIG. 6B.

### (2) Determination of Presence or Absence of Mutation

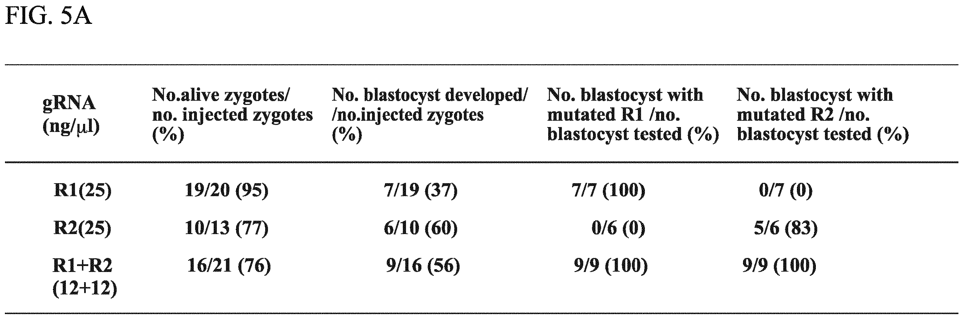

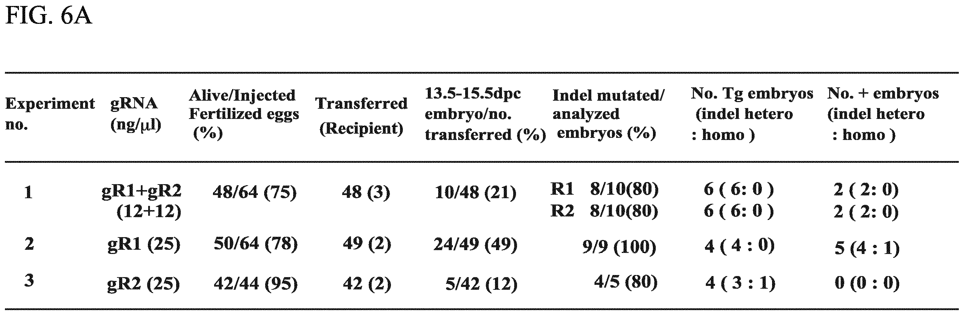

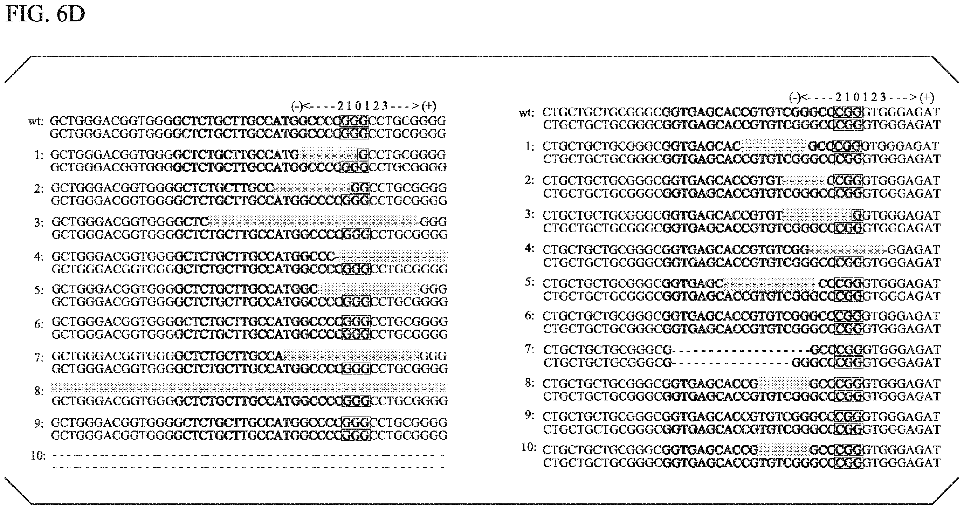

A nuclear extract of one embryo of the blastocysts or a DNA solution prepared from the tail or the hand of the embryos 13.5 days after the implantation was subjected to PCR using a Ramp 1-2S primer and a Ramp1-2A primer, and a Ramp2-5S primer and a Ramp 2-6A, and therefore a PCR product was obtained. Subsequently, the PCR product was sequenced using BigDye Terminator v.3.1 and ABI Genetic Analyzer 3130 Ramp1. The presence or absence of mutation of Ramp 1 gene and Ramp2 gene was determined. The results are shown in FIG. 5A (blastocysts), and in FIGS. 6A and 6D (embryos 13.5 days after the implantation).

### (3) T7EI Assay

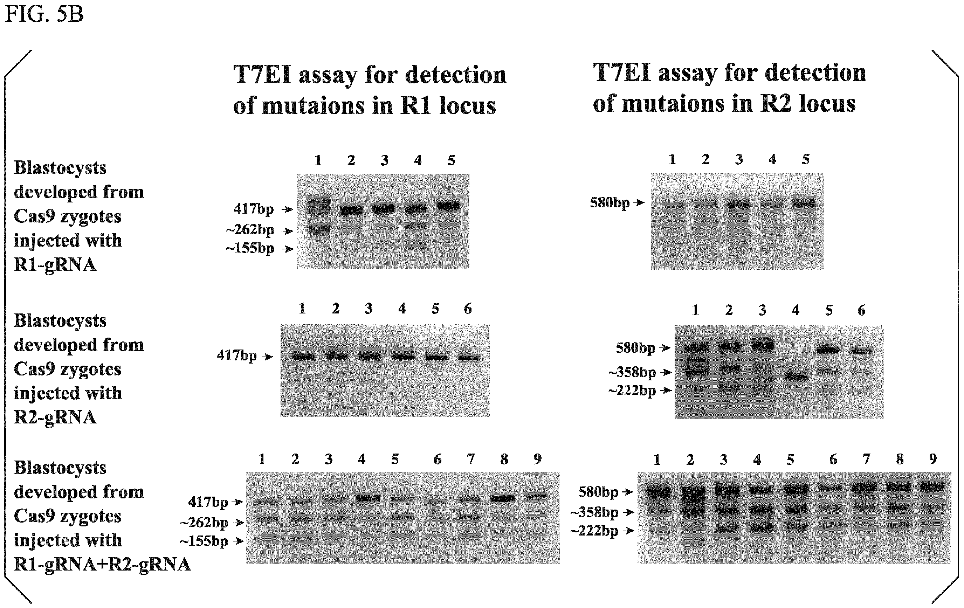

The nuclear extract of one embryo of the blastocysts or the DNA solution prepared from the tail or the hand of the embryos 13.5 days after the implantation was subjected to T7EI Assay using the same method as in (3) of Test Example 2. For blastocysts into which R1-gRNA was introduced, a Ramp1-2S primer and a Ramp1-2A primer were used. The results are shown in FIG. 5B (blastocysts) or FIG. 6C (embryos 13.5 days after the implantation).

Based on FIGS. 5A and 5B, in a case where only R1-gRNA was injected into the fertilized egg, the indel mutation was recognized only at the Ramp1 locus. The same results were also confirmed in a case where only R2-gRNA was injected into the fertilized egg. In a case where R1-gRNA and R2-gRNA were injected at the same time, simultaneous mutagenesis was observed at both loci.

Based on the above results, it could be confirmed by the blastocyst that genomic editing in the fertilized egg injected only with the guide RNA works specifically at the target locus.

Furthermore, when the indel mutation in the stage of embryos derived from the fertilized egg (Tg/+ female mouse and +/+ male mouse) injected with R1-gRNA and R2-gRNA at the same time was examined, almost all of the embryos were hetero- or homo-genetically modified embryos (refer to FIG. 6A).

In addition, the presence or absence of mutation at each of the three types of off-target sites predicted in each of the Ramp1 gene and Ramp2 gene was examined by subjecting a DNA solution prepared from the tail or the hand of the obtained embryos of ten pups (using Experiment 1 shown in FIG. 6A) to PCR using a primer for off-target analysis shown in the above table. As a result, not one mutation was recognized.

### [Test Example 4] Comparative Examination 1 with Method of the Related Art

Subsequently, a genomic editing method for microinjecting synthetic Cas9 mRNA and guide RNA into a fertilized egg, and a genomic editing method by maCas9 of the present invention were compared.

### (1) Introduction of Guide RNA

Fertilized eggs containing maCas9 were produced by artificial fertilization using Tg/+ egg cell and +/+ sperm. Subsequently, only R2-gRNA (25 ng/µL) was microinjected into the cytoplasm and the pronucleus of the fertilized eggs containing maCas9 (genotype: Tg/+ or +/+). As a control group, both synthetic Cas9 mRNA (50 ng/µL) and R2-gRNA (25 ng/µL) were injected into the cytoplasm and the pronucleus of the wild-type fertilized eggs (not containing maCas9; genotype +/+). These fertilized eggs were cultured to blastocysts.

### (2) Determination of Genotype and Presence or Absence of Mutation

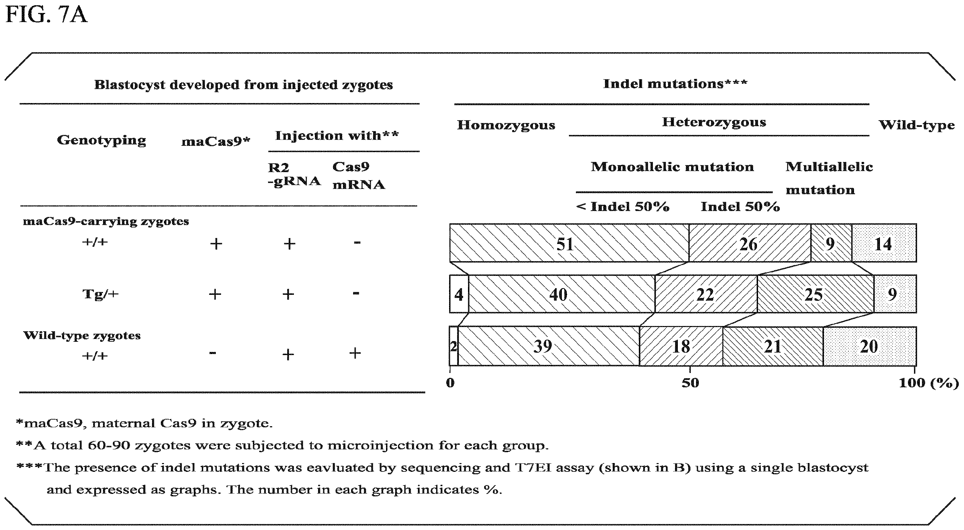

A genotype was determined by using the same method as in (3) of Example 1. Furthermore, the presence or absence of mutation was determined by using the same method as in (2) of Test Example 3. The results are shown in FIG. 7A.

### (3) T7EI Assay

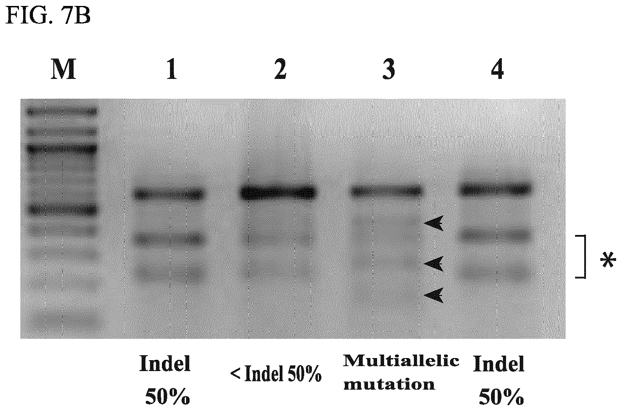

T7EI assay was carried out on the blastocysts having a heterozygous mutant (monoallelic or multiallelic) using the same method as in (3) of Test Example 2. The results are shown in FIG. 7B.

In FIG. 7B, "<IndeI 50%" in lane 2 indicates that the total intensity of two cleaved bands is smaller than that of a parent band. In addition, in "Indel 50%" in lanes 1 and 4, the total intensity of two cleaved bands is almost the same as a parent band. In "Multiallelic mutation" in lane 3, two or more cleaved bands are generated.

Based on FIG. 7A, induction rates of the mutation at the target site and the indel mutant types (homozygous (biallelic), monoallelic, or multiallelic KO) were statistically equivalent to three experimental groups (maCas9-carrying +/+ and Tg/+ fertilized eggs, and a control group (maCas9-non-carrying +/+ fertilized egg)) (p> 0.05, oneway-ANOVA). In addition, nonmosaic variants (50% indel mutation) and mosaic variants (<50% monoallelic indel mutation and multiallelic mutation) in the maCas9-carrying +/+ and Tg/+ fertilized eggs were also statistically equivalent to those in the control group (p> 0.05, oneway-ANOVA). Furthermore, it was recognized that an induction rate of the multiallelic mosaic variants in the maCas9-carrying +/+ fertilized eggs tended to be lower compared to that of the maCas9-carrying Tg/+ fertilized eggs and the control group, but an induction rate of the nonmosaic variants thereof (50% indel mutation) tended to be higher.

In addition, the presence or absence of base sequence mutation at the three types of the predicted off-target sites in blastocysts generated from maCas9-carrying fertilized eggs injected with R2-gRNA was examined by PCR using a primer for off-target analysis shown in the above table. As a result, there was no mutation even at one location.

Based on the above result, the genomic editing ability by maCas9 is considered to be equivalent to that in the method for injecting synthetic Cas9 into fertilized eggs. In addition, it became clear that in the genomic editing by maCas9, the mosaic variants significantly reduced compared with the method for injecting synthetic Cas9 into fertilized eggs.

### [Test Example 5] Confirmation Test of Simultaneous Generation of Multiple Gene Mutations

Subsequently, the number of mutations which can be induced simultaneously in embryos in which maCas9 is not present in the Cas9 transgene was examined.

### (1) Introduction of Guide RNA

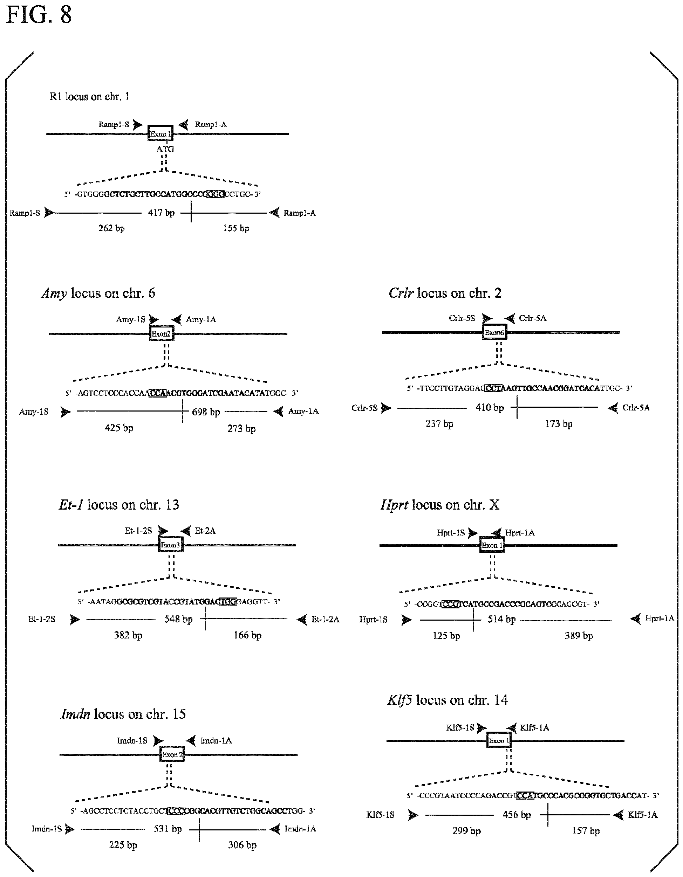

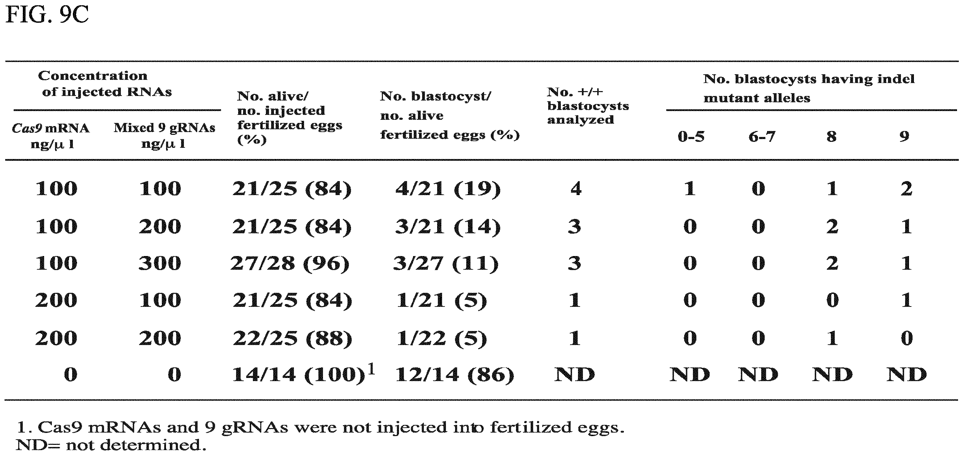

Nine different types of guide RNAs were microinjected into fertilized eggs prepared by in vitro fertilization of a Tg/+ female mouse and a +/+ male mouse. The target genes are nine types, which are Ramp1 gene, Ramp2 gene, Amy gene, Crlr gene, Et-1 gene, Hprt gene, Imdn gene, and Klf5 gene. The sequence of the guide RNA is as shown in "Guide RNA" in the above table, and FIGS. 4C and 8. As a control group, these nine types of the guide RNAs and synthetic Cas9 mRNA (refer to FIG. 9C) were microinjected into fertilized eggs prepared by in vitro fertilization of a +/+ female mouse and a +/+ male mouse.

### (2) Determination of Genotype and Presence or Absence of Mutation

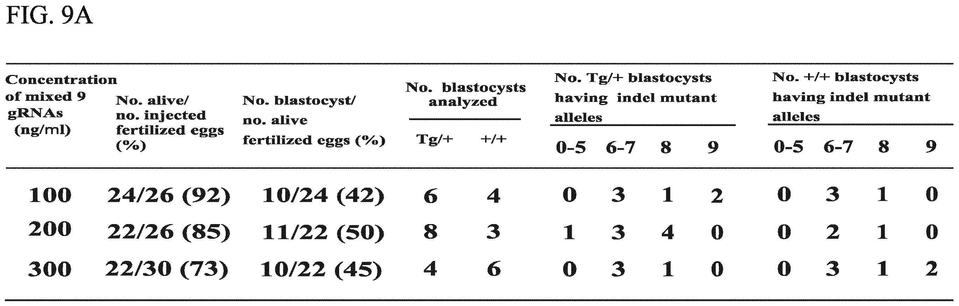

A genotype was determined by using the same method as in (3) of Example 1. Furthermore, the presence or absence of mutation was determined by using the same method as in (2) of Test Example 3. As a primer at this time, the Amy-1S primer and the Amy-1A primer, the Crlr-5S primer and the Crlr-5A primer, the Et-1-1S primer and the Et-1-1A primer, the Hprt-1S primer and the Hprt-1A primer, the Imdn-1S primer and the Imdn-1A primer, the Klf5-1S primer and the Klf5-1A primer, the Ramp1-2S primer and the Ramp1-2A, and the Ramp2-5S primer and the Ramp 2-6A, which are described in the above tables <Primer>, were used. The results are shown in FIG. 9A.

### (3) T7EI Assay

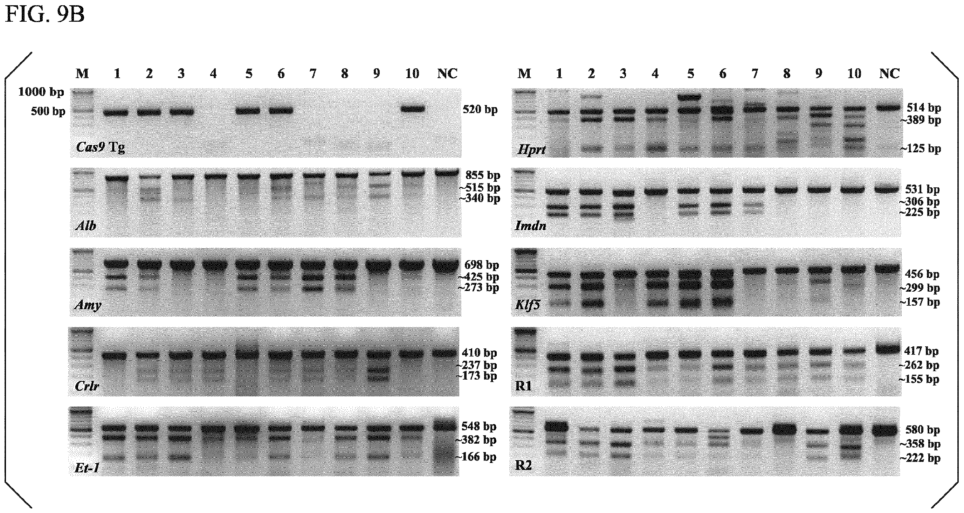

T7EI Assay was carried out using the same method as in (3) of Test Example 2. The results are shown in FIG. 9B. A primer used is the same as that exemplified in (2) of the present test example.

Based on FIGS. 9A and 9C, it could be confirmed that in the blastocysts using the method of the present invention, indel mutations were simultaneously generated at an average of 7.3 loci, which was equivalent to that of the control group. In addition, two cases of each of nine types of simultaneous indel mutations were observed in the blastocysts derived from the maCas9-carrying +/+ and Tg/+ fertilized eggs.

Furthermore, importantly, a development rate of the blastocyst using the method of the present invention was 42% to 50%, but a development rate of the blastocyst of the control group was 5% to 19%, which was extremely low to obtain the pup.

Based on the above result, it can be said that the fertilized eggs containing maCas9 can be simultaneously introduced with mutations into multilocus and thus are extremely useful for the production of a genetic modification mouse having multigene.

### [Test Example 6] In Vitro Genetic Modification Test

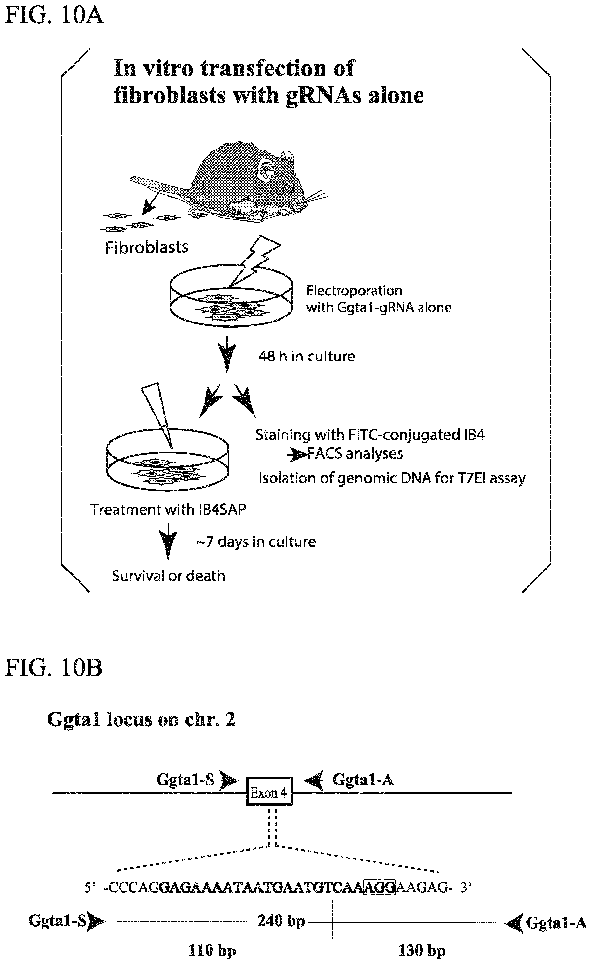

Next, in order to verify the practicality of the Cas9 mouse in in vitro genetic modification, disruption of Ggat1 gene involved in synthesis of α-Gal epitope of the cell surface was investigated. FIG. 10A shows a protocol in the present test.

### (1) Introduction of guide RNA

Fibroblasts were primarily cultured from the tails of a Tg/+ and +/+ pup of the Cas9 mouse. The culturing was carried out for 5 days using DMEM (Dulbecco's modified Eagle's essential medium) containing 10% fetal bovine serum. Subsequently, 100 µg/mL of Ggta1 gRNA(SEQ ID NO: 22) dissolved in phosphate-buffered saline (calcium and magnesium-free, pH 7.4) was transiently introduced into 5 × 10⁶ cultured fibroblasts by an electroporation method using Gene-Pulser II (Manufactured by Bio-Rad Laboratories, Inc.). Abase sequence homologous to the Ggat1 gene in Ggta1 gRNAis shown in FIG. 10B.

### (2) T7EI assay

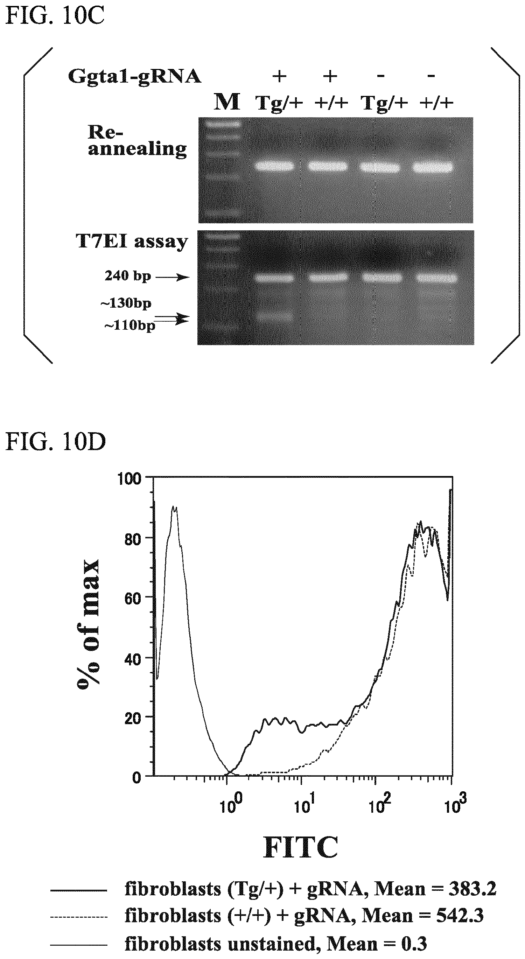

A PCR product was prepared by PCR from a homogenate of the fibroblasts after introduction of the guide RNA by using the Ggta1-S primer and the Ggta1-A primer. Subsequently, T7EI Assay was carried out using the same method as in (3) of Test Example 2. The results are shown in FIG. 10C.

Based on FIG. 10C, it was confirmed that the indel mutation occurred only in the Ggta1 gene.

### (3) FACS Analysis

By using the fibroblasts after introduction of the guide RNA, FACS analysis was carried out by utilizing the fact that FITC-labeled isolectin, BS-I-B4 (IB4) specifically recognizes α-Gal epitope. The results are shown in FIG. 10D.

Based on FIG. 10D, a decrease in α-Gal epitope expression (up to 30% reduction compared to a wild-type cell), which was the target, was confirmed.

### (4) Survival Assay

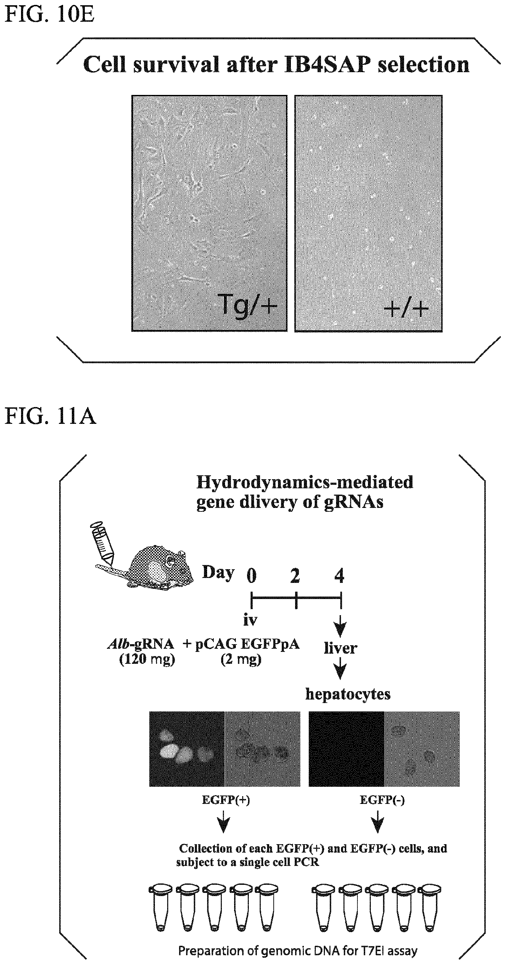

The fibroblasts after introduction of the guide RNA were treated with IB4-saporin conjugate (IB4SAP). The death of cells having 2 genes of Ggta1 (+/+ type) and cells having 1 gene knocked out of Ggta1 (KO/+ type) was recognized. On the other hand, survival of cells considered to be biallelic Ggta1 KO was observed (refer to FIG. 10E). The survival rate was 20 (colony) per 5 × 10⁵ cells, and an induction efficiency of gene defect estimated from the above rate was estimated to be 0.004%.

Based on the above result, it was confirmed that the primary cultured cells prepared from the Cas9 mouse line caused genomic editing by CRISPR/Cas9 only by introducing the guide RNA.

### [Test Example 7] In Vivo Genetic Modification

To verify that in vivo genomic editing is possible by administration of the guide RNA in the Cas9 mouse, disruption of genes of hepatocytes by a hydrodynamic guide RNA introduction method was investigated. FIG. 11A shows a protocol in the present test.

### (1) Introduction of Guide RNA

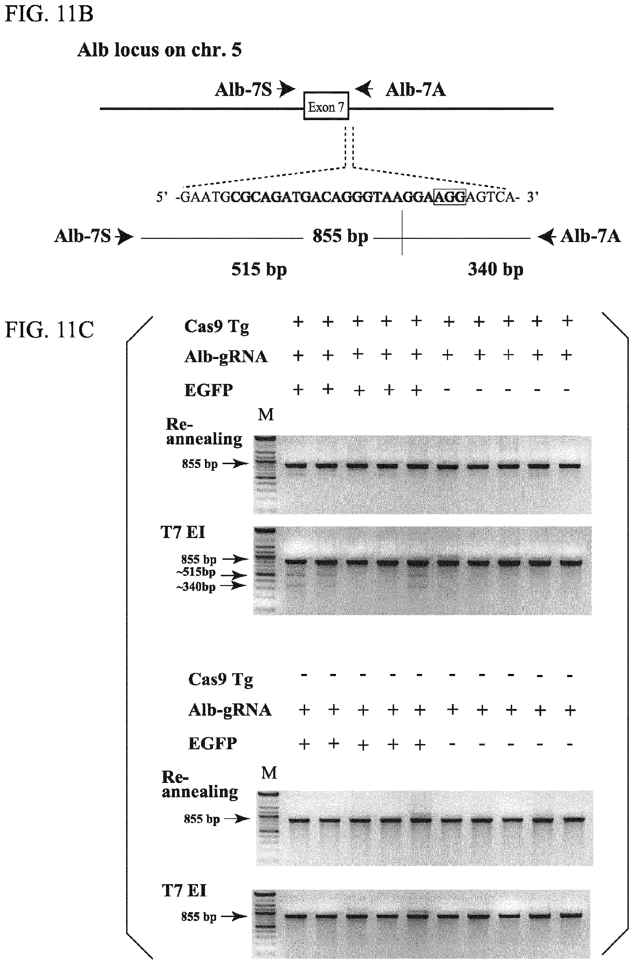

120 µg of albumin (Alb) gRNA (SEQ ID NO: 18) and 4 µg of pCAG-EGFPpA were injected simultaneously into the tail vein of the Cas9 mouse and the wild-type mouse by using TransIT-QR Hydrodynamic Delivery Solution kit (manufactured by Mirus Bio LLC). A base sequence homologous to Alb1 gene in Alb gRNA is shown in FIG. 11B.

Thereafter, the hepatocytes were isolated from each mouse. By this method, EGFP-positive cells were obtained at a ratio of 0.1% (1/1000 cells).

### (2) T7E1 Assay

30 EGFP-positive cells and 30 EGFP-negative cells were isolated from the hepatocytes derived from the Cas9 mouse. Similarly, 30 EGFP-positive cells and 30 EGFP-negative cells were isolated from the hepatocytes derived from the wild-type mouse. Subsequently, a PCR product was prepared by PCR from a homogenate of each cell by using the Alb-7S primer and the Alb-7A primer. Subsequently, T7EI Assay was carried out using the same method as in (3) of Test Example 2 so as to examine the presence or absence of indel mutation. The results are shown in FIG. 11C.

Based on FIG. 11C, the indel mutation of Alb1 gene was recognized only in the hepatocytes derived from the EFGP-positive-Cas9 mouse at a rate of about 50% (15/30), and a ratio of the hepatocytes in which the mutation occurred was calculated to be 0.05% (1/2000 cells). In the hepatocytes derived from the EGFP-negative-Cas9 mouse, the hepatocytes derived from the EGFP-positive-wild type mouse, and the hepatocytes derived from the EGFP-negative-wild type mouse, the indel mutation of Alb1 gene was not recognized.

Based on the above results, it became clear that in vivo administration of the guide RNA causes the indel mutation of the endogenous target gene of the Cas9 mouse.

### [Test Example 8] Comparative Examination 2 with Method of the Related Art

Based on Test Example 4, it was shown that the genomic editing ability by maCas9 was equivalent to that in the method for injecting synthetic Cas9 mRNA into fertilized eggs by the injection of Ramp2 gRNA into the fertilized egg and its analysis at the blastocyst stage, and it was shown that an induction rate of the multiallelic mosaic variants in the maCas9-carrying +/+ fertilized eggs tended to be lower compared to that of the maCas9-carrying Tg/+ fertilized eggs and control group. In order to more strongly back up this finding, ET-1-gRNA as gRNA of another gene was introduced into fertilized eggs, and these fertilized eggs were developed up to embryos of 12.5 to 13 days old, and an indel mutation ratio and a mosaic ratio were examined.

### (1) Introduction of Guide RNA

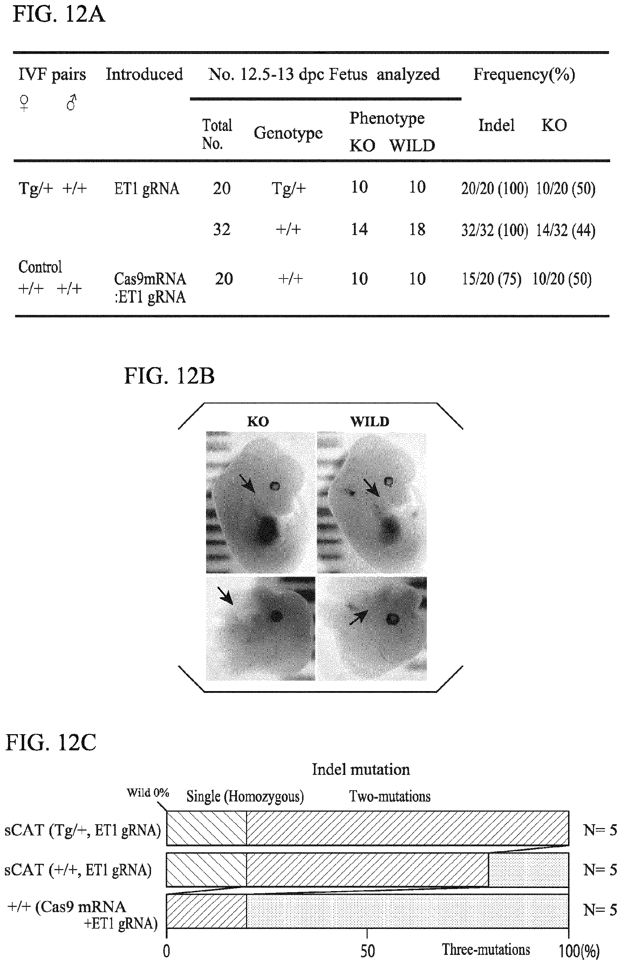

Fertilized eggs containing maCas9 were produced by artificial fertilization using Tg/+ egg cell and +/+ sperm. Subsequently, only ET-1-gRNA (200 ng/µL) was introduced into the cytoplasm and the pronucleus of the fertilized eggs containing maCas9 (genotype: Tg/+ or +/+) by an electroporation method. As a control group, both synthetic Cas9 mRNA (100 ng/µL) and ET-1-gRNA (200 ng/µL) were introduced into the wild-type fertilized eggs (not containing maCas9; genotype +/+) by using the electroporation method. These fertilized eggs were cultured to blastocysts. Each of the blastocysts into which ET-1-gRNA was introduced was implanted into the uterus of an ICR strain pseudopregnant female mouse. 12.5 to 13 days after implantation, embryos were obtained. The form of embryos of ten pups 12.5 to 13 days after the implantation is shown in FIG. 12B.

### (2) Determination of Genotype and Presence or Absence of Mutation

A genotype was determined by using the same method as in (3) of Example 1. Furthermore, the presence or absence of mutation was determined by using the same method as in (2) of Test Example 3 except that the Et-1-1S primer and the Et-1-1A primer were used. Furthermore, fetuses of the Et-1 KO phenotype (each N = 5) among Tg/+ and +/+, and the control group were randomly selected to be analyzed, and therefore a mosaic ratio was calculated. The results are shown in FIG. 12C.

Based on FIG. 12A, with respect to 75% in the control group, the indel mutation was 100% in the Tg/+ and +/+ fetuses of the Cas9 mouse, which was the high ratio.

In addition, based on FIG. 12B, abnormality of jaw formation was observed in the Et-1 knockout (KO) phenotype.

Based on FIG. 12A, with respect to 50% in the control group, this Et-1 KO phenotype was 50% and 44% in each of the Tg/+ and +/+ fetuses of the Cas9 mouse.

In addition, based on FIG. 12C, in regard to a mosaic ratio, there were mostly two types of the indel mutations in the Tg/+ and +/+ fetuses of the Cas9 mouse, but one type (homozygous: only the same mutant genome) was obtained. On the other hand, in the control group, there were mostly three types.

Based on the above results, it was shown that the genomic editing ability by maCas9 was equivalent to that in the method for injecting synthetic Cas9 mRNA into fertilized eggs, but a mosaic ratio was lower compared to that of the method for injecting Cas9 mRNA/gRNA of the related art, and accordingly, characteristics of the genomic editing ability by maCas9 (high efficiency and low mosaic ratio) were again verified in the Et-1 gene.

### [Test example 9] Test of Examination on gRNA Introduction Timing

The influence on the indel ratio and the mosaic ratio by a difference in timing of gRNA introduction into the fertilized egg derived from Cas9 mouse was examined.

### (1) Introduction of Guide RNA

Fertilized eggs containing maCas9 were produced by artificial fertilization using Tg/+ egg cell and +/+ sperm. Subsequently, only ET-1-gRNA (200 ng/µL) was introduced into the cytoplasm and the pronucleus of the fertilized eggs containing maCas9 (genotype: Tg/+ or +/+) after 6 to 8 hours, or 10 to 12 hours from insemination, by an electroporation method. These fertilized eggs were cultured to blastocysts.

### (2) Determination of Genotype and Presence or Absence of Mutation

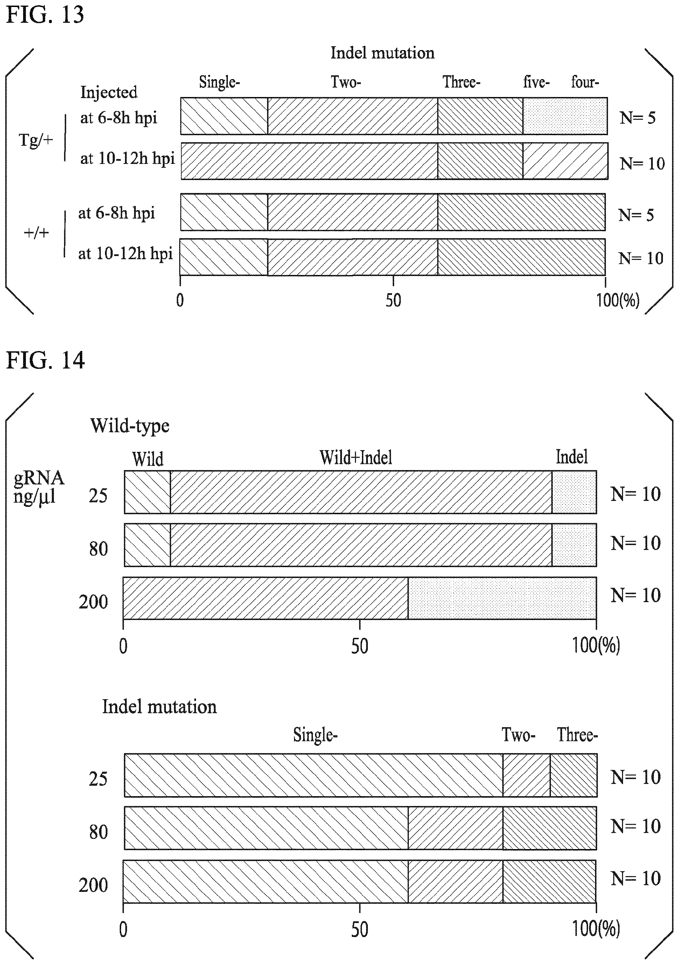

A genotype was determined by using the same method as in (3) of Example 1. Furthermore, the presence or absence of mutation was determined by using the same method as in (2) of Test Example 3 except that the Et-1-1S primer and the Et-1-1A primer were used. Furthermore, fetuses of the Et-1 KO phenotype (each N = 5) among Tg/+ and +/+ were randomly selected to be analyzed, and therefore a mosaic ratio was calculated. The results are shown in FIG. 13.

Based on FIG. 13, in any of the fertilized eggs into which gRNA was introduced after 6 to 8 hours, and 10 to 12 hours from insemination, the indel ratio was high (100% in any egg).

In the Tg/+ blastocysts, in comparison under the conditions after 6 to 8 hours, and 10 to 12 hours from insemination, a ratio of a single indel genomic mutation decreased under the condition after 10 to 12 hours, and an increase of multiallelic mosaic mutation was recognized.

On the other hand, in the +/+ blastocysts under the conditions after 6 to 8 hours, and 10 to 12 hours from insemination, no change was recognized in ratios of a single indel genomic mutations and multiallelic mosaic mutation. This was presumed to be due to the fact that gRNA introduced in the later stage exists longer without degradation than the gRNA introduced in the early stage. Furthermore, from the fact that the genotype is Tg/+ embryo, a possibility that Cas9 derived from zygote was transcribed and translated to work was considered.

Based on the above result, a change in the number of indel genomic mutation due to the timing of gRNA introduction into the Tg/+ fertilized egg was recognized. In order to reduce the multiallelic mosaic mutation, it was suggested that it is preferable to introduce gRNA into the fertilized egg at the early stage. On the other hand, it was suggested that for the purpose of obtaining an individual having many multiallelic mosaic mutations, the fertilized egg of the late stage is preferably utilized.

### [Test Example 10] Test of Examination on gRNA concentration

The influence on the indel mutation ability by a change in concentration of introduced gRNA was examined.

### (1) Introduction of Guide RNA

Fertilized eggs containing maCas9 were produced by artificial fertilization using Tg/+ egg cell and +/+ sperm. Subsequently, at three different concentrations of 25 ng/µL, 80 ng/µL, and 200 ng/µL, only ET-1-gRNA was introduced into the cytoplasm and the pronucleus of the fertilized eggs containing maCas9 (genotype: Tg/+ or +/+) 6 to 8 hours from insemination by using an electroporation method. These fertilized eggs were cultured to blastocysts.

### (2) Determination of Genotype and Presence or Absence of Mutation

A genotype was determined by using the same method as in (3) of Example 1. Furthermore, the presence or absence of mutation was determined by using the same method as in (2) of Test Example 3 except that the Et-1-1S primer and the Et-1-1A primer were used. Furthermore, fetuses of the Et-1 KO phenotype (each N = 5) among Tg/+ and +/+ were randomly selected to be analyzed, and therefore a mosaic ratio was calculated. The results are shown in FIG. 14.

Based on Fig. 14, the indel mutation was 90% to 100%, which is a high ratio, under the conditions at any concentration of 25 ng/µL, 80 ng/µL, and 200 ng/µL. The non-indel mutation (wild) was not recognized under the condition of 200 ng/µL ET-1-gRNA introduction, and the embryo of only the indel mutant genome reached up to 40%.

In addition, it was recognized that the number of the indel mutation per embryo increased concentration-dependently. That is, under the condition of 25 ng/µL ET-1-gRNA introduction, 80% was one type of the indel mutation (including homozygous (only the same mutant genome) and heterozygous (only a wild-type and one type of indel mutant genomes)). On the other hand, under the conditions of 80 ng/µL and 200 ng/µL ET-1-gRNA introductions, the indel mutation ratio decreased down to 60% and 20%, respectively.

In addition, an increase of two types of the indel mutations (including only two types of the indel mutant genomes, and a wild-type and two types of indel mutant genomes) was recognized.

Based on the above results, it became clear that it is possible to change the number of embryo indel mutant genomes by changing a concentration of gRNA to be introduced.

In the condition at a low concentration (25 ng/µL), most were embryos including a wild-type and one type of indel mutant genomes. These are considered to be advantageous characteristics in a case where homozygosity is generated, in a case where it is desired to obtain a genetic modification individual by avoiding lethal mutation, and in a case where reversely, a heterozygous mutant individual is preferentially desired.

### [Test Example 11] Test of Knockin of Foreign Gene to Klf5 Gene

Whether knockin of a foreign gene is also possible in the fertilized egg derived from the Cas9 mouse was verified by a test of knockin of a foreign gene to Klf5 gene.

### (1) Design of Guide RNA and Foreign Gene (KI Fragment)

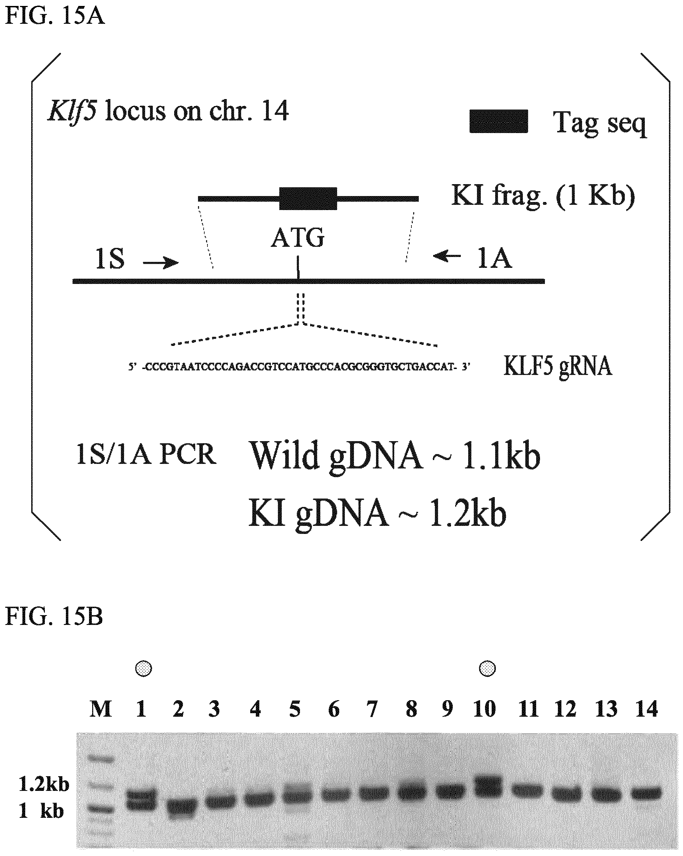

As shown in FIG. 15A, gRNA was designed near the atg base for the translation initiation of the Klf5 gene (Klf5 gRNA2: SEQ ID NO: 79). Furthermore, to the region thereof in the 5' and 3' directions, about 100 bp of foreign genes (a total of about 1 kb) containing a Flag-tagged sequence in which about 500 bp of homologous DNA sequences were linked were synthesized as a knockin sequence (KI fragment) (KI Fragment: SEQ ID NO: 80).

### (2) Introduction of Guide RNA

Fertilized eggs containing maCas9 were produced by artificial fertilization using Tg/+ egg cell and +/+ sperm. In addition, fertilized eggs of a control group were produced by artificial fertilization using +/+ egg cell and +/+ sperm. Subsequently, KIf5 gRNA2 (10 ng/µL) produced in (1) and the KI fragment (3.3 ng/µL) produced in (1) were injected into the cytoplasm and the pronucleus of the fertilized eggs containing maCas9 (genotype: Tg/+ or +/+) 10 to 12 hours after insemination. In addition, KIf5 gRNA2 (10 ng/µL) produced in (1) and the KI fragment (3.3 ng/µL) produced in (1), and Cas9 mRNA (33 ng/µL) were injected into the cytoplasm and the pronucleus of the fertilized eggs of the control group of the same stage. These fertilized eggs were cultured to blastocysts.

### (2) Determination of Genotype and Presence or Absence of Mutation

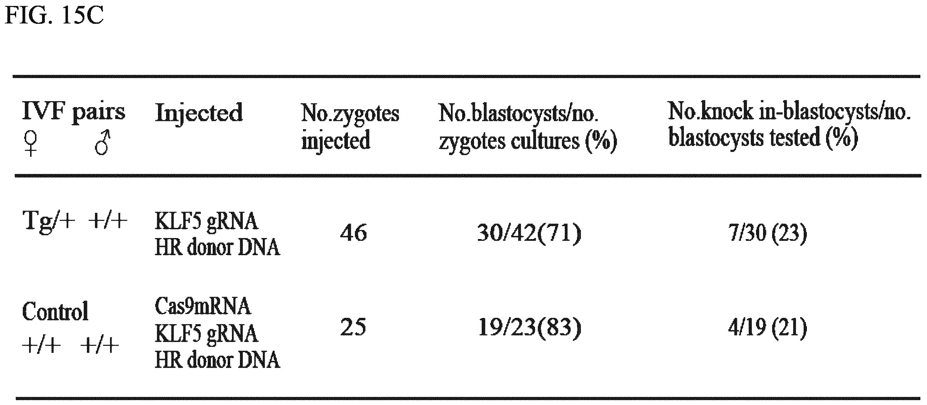

A genotype was determined by using the same method as in (3) of Example 1. In addition, a solution for PCR was prepared from each blastocyst and PCR was carried out by using PCR primers (KlfS-1S primer (SEQ ID NO: 52) and Klf5-1A primer (SEQ ID NO: 53)) set in a genomic DNA sequence outside of a homologous sequence of the KI fragment. The knockin-Klf5 gene was determined from the presence or absence of a PCR product (about 1.2 kb) having a size larger by about 0.1 kb than a wild-type PCR product (about 1.1 kb). The results are shown in FIG. 15B and FIG. 15C.

Based on FIG. 15B, the PCR product larger than the wild-type PCR product of about 1.2 kb was detected in Samples No. 1 and No. 10.

In addition, based on FIG. 15C, 7 embryos among 30 embryos of blastocysts derived from the fertilized eggs of the Cas9 mouse were knockin-embryos (23%). On the other hand, 4 embryos among 19 embryos of blastocysts derived from the fertilized eggs of the control group were knockin-embryos (21%).

Based on the above results, it was possible to knockin the foreign genes by using the fertilized eggs containing maCas9, and it became clear that the efficiency thereof was equivalent to that in the method for injecting Cas9 mRNA/gRNA of the related art.

### [Example 2] Establishment of Mouse Line Expressing Nickase Cas9 (D1OA) in Its Entirety

In addition to the characteristics of the Cas9 mouse described above, establishment of a Nickase Cas9 (D10A) mouse, which is a transgenic mouse which has both a processing ability for reducing an off-target rate and a processing ability of Nick genome, and in which NickaseCas9 is expressed in its entirety, was carried out.

### (1) Design of Construct for Inserting Cas9 Gene

Using, as a template, pCAG-NFCas9pA and pCAG-FCas9pA constructed in Example 1, PCR was carried out by using A NFL (D10A) S primer (SEQ ID NO: 81) and a NFL (D10A) A primer (SEQ ID NO: 82) so as to construct a Cas9 (D10A) vector in which the 10^{th} amino acid of Cas9 was substituted with A from D.

### (2) Injection into Fertilized Mouse Egg

A fertilized egg to be used was produced by fertilizing sperm of a male B6 mouse and an egg cell of a female BDF1 mouse by using a general in vitro fertilization (IVF) method.

Subsequently, an injection solution was prepared so that each of the two types of Cas9 (D10A) vectors prepared in (1) became 2 ng/µL. Subsequently, the injection solution was injected into the pronuclei of the fertilized mouse eggs. Subsequently, the fertilized egg into which the gene was introduced was implanted into the oviduct of an ICR pseudopregnant female mouse, and therefore F1 generation of the Nickase Cas9 (D10A) transgenic mouse was obtained.

### (3) Determination of Genotype

A genotype of 30 grown pups of the F1 generation was determined by using the same method as in (3) of Example 1. Identification of introduction of Cas9 (D10A) was carried out by PCR using a CAG1620 primer (SEQ ID NO: 32) and a Cas9 215-A primer (SEQ ID NO: 34). As a result, 6 pups were Tg individuals. In regard to the above 6 pups, no influence on development and survival was recognized at the present time, as in the case of the Cas9 mouse.

In addition, by continuing crossing among the above 6 pups, a Nickase Cas9 (D10A) mouse of 2 lines was established. When cDNA was prepared from major organs such as the lung and ovary of the pup of the Nickase Cas9 (D10A) mouse of 2 lines so as to examine the Cas9 (D10A) expression by Real Time PCR using the cDNA, the Cas9 (D10A) expression was confirmed.

### Industrial applicability

According to the method for modifying a target gene of the present invention, it is possible to edit a plurality of different genes or a plurality of locations in the same gene at the same time with high efficiency. In addition, according to the method for modifying a target gene of the present invention, a genetic modification non-human organism can be rapidly produced at a low cost. Furthermore, the method for modifying a target gene of the present invention can be utilized in studies of lifestyle diseases such as cancer or heart disease in which abnormality of many genes is involved, or disease-related genes identified by genome-wide association study (GWAS).

## Claims

1. Female reproductive cells derived from a genetically modified non-human mammal **characterized in that** a protein or mRNA of maternally derived Cas9 accumulates in the female reproductive cells, wherein the genetically modified non-human mammal has a nuclear genome into which at least 3 copies of genes that code for Cas9 (CRISPR-associated 9) are introduced and wherein the genetically modified non-human mammal is one selected from the group consisting of a mouse, rat, hamster, guinea pig, rabbit, dog, cat, horse, cow, sheep, pig, goat, marmoset, and monkey.

2. The female reproductive cells according to Claim 1, wherein the nucleus of the female reproductive cells, does not contain the genes that code for the Cas9

3. The female reproductive cells according to Claim 1 or 2, wherein the Cas9 is a variant.

4. The female reproductive cells according to Claim 3, wherein the Cas9 is a variant lacking activity of cleaving one or both strands of a target gene containing a target sequence.

5. The female reproductive cells according to any one of Claims 1 to 4, wherein the genes that code for the Cas9 are tandemly introduced into the nuclear genome.

6. The female reproductive cells according to any one of Claims 1 to 5, wherein the genetically modified non-human mammal is one selected from the group consisting of a mouse, rat, hamster, guinea pig, rabbit, dog, cat, horse, cow, sheep, pig, and goat.

7. A method for modifying target genes, comprising a step of introducing a guide RNA into female reproductive cells of any one of Claims 1 to 6.

8. A method for modifying target genes, comprising:
- a step of introducing a guide RNA into female reproductive cells of any one of Claims 1 to 3, 5 or 6,
- a step of cleaving the target genes at a cleavage site located upstream of a PAM (proto-spacer adjacent motif) sequence by Cas9 expressed in the female reproductive cells, and
- a step of obtaining the modified target genes in a region determined by complementary binding of the guide RNA and the target genes,
wherein the target genes have the PAM sequence, and the guide RNA contains a polynucleotide including a base sequence complementary to an upstream base sequence of the PAM sequence in the target genes.

9. A method for modifying target genes, comprising:
- a step of introducing a guide RNA into female reproductive cells of any one of Claims 1 to 3, 5 or 6,
- a step of cleaving the target genes at a cleavage site located three bases upstream of a PAM sequence by Cas9 expressed in the female reproductive cells, and
- a step of obtaining the modified target genes in a region determined by complementary binding of the guide RNA and the target genes,
wherein the target genes have the PAM sequence including NGG (N is any one base selected from the group consisting of adenine, cytosine, thymine, and guanine), and the guide RNA contains a polynucleotide including base sequences complementary to base sequences from one base upstream to 20 bases to 24 bases upstream of the PAM sequence in the target genes.

## Patentansprüche

1. Weibliche Fortpflanzungszellen, die von einem genetisch modifizierten nichtmenschlichen Säuger stammen, **dadurch gekennzeichnet, dass** ein Protein oder eine mRNA von mütterlich abstammendem Cas9 in den weiblichen Fortpflanzungszellen akkumuliert ist, wobei der genetisch modifizierte nicht-menschliche Säuger ein Kerngenom aufweist, in das mindestens 3 Kopien von Genen, die für Cas9 (CRISPRassoziiertes 9) kodieren, eingeführt sind, und wobei der genetisch modifizierte nicht-menschliche Säuger einer ist, der aus der Gruppe ausgewählt ist, die aus Maus, Ratte, Hamster, Meerschweinchen, Kaninchen, Hund, Katze, Pferd, Kuh, Schaf, Schwein, Ziege, Marmoset und Affe besteht.

2. Weibliche Fortpflanzungszellen nach Anspruch 1, wobei der Kern der weiblichen Fortpflanzungszellen nicht die Gene enthält, die für das Cas9 kodieren.

3. Weibliche Fortpflanzungszellen nach Anspruch 1 oder 2, wobei es sich bei dem Cas9 um eine Variante handelt.

4. Weibliche Fortpflanzungszellen nach Anspruch 3, wobei es sich bei dem Cas9 um eine Variante handelt, der die Aktivität fehlt, einen oder beide Stränge eines Zielgens, das eine Zielsequenz enthält, zu spalten.

5. Weibliche Fortpflanzungszellen nach einem der Ansprüche 1 bis 4, wobei die Gene, die für das Cas9 kodieren, tandemartig in das Kerngenom eingeführt sind.

6. Weibliche Fortpflanzungszellen nach einem der Ansprüche 1 bis 5, wobei der genetisch modifizierte nicht-menschliche Säuger einer ist, der aus der Gruppe ausgewählt ist, die aus Maus, Ratte, Hamster, Meerschweinchen, Kaninchen, Hund, Katze, Pferd, Kuh, Schaf, Schwein und Ziege besteht.

7. Verfahren zur Modifizierung von Zielgenen, umfassend einen Schritt der Einführung einer Leit-RNA in weibliche Fortpflanzungszellen nach einem der Ansprüche 1 bis 6.

8. Verfahren zur Modifizierung von Zielgenen, umfassend
- einen Schritt der Einführung einer Leit-RNA in weibliche Fortpflanzungszellen nach einem der Ansprüche 1 bis 3, 5 oder 6,
- einen Schritt der Spaltung der Zielgene an einer stromaufwärts von einer PAM-Sequenz (proto-spacer adjacent motif) gelegenen Spaltstelle durch Cas9, das in den weiblichen Fortpflanzungszellen exprimiert wird, und
- einem Schritt der Gewinnung der modifizierten Zielgene in einem Bereich, der durch komplementäre Bindung der Leit-RNA und der Zielgene bestimmt wird,
wobei die Zielgene die PAM-Sequenz aufweisen und die Leit-RNA ein Polynukleotid enthält, das eine Basensequenz enthält, die komplementär zu einer stromaufwärts gelegenen Basensequenz der PAM-Sequenz in den Zielgenen ist.

9. Verfahren zur Modifizierung von Zielgenen, umfassend
- einen Schritt der Einführung einer Leit-RNA in weibliche Fortpflanzungszellen nach einem der Ansprüche 1 bis 3, 5 oder 6,
- einen Schritt der Spaltung der Zielgene an einer Spaltstelle, die sich drei Basen stromaufwärts von einer PAM-Sequenz befindet, durch Cas9, das in den weiblichen Fortpflanzungszellen exprimiert wird, und
- einen Schritt der Gewinnung der modifizierten Zielgene in einem Bereich, der durch komplementäre Bindung der Leit-RNA und der Zielgene bestimmt wird,
wobei die Zielgene die PAM-Sequenz aufweisen, die NGG einschließt (N ist eine beliebige Base, ausgewählt aus der Gruppe, die aus Adenin, Cytosin, Thymin und Guanin besteht), und die Leit-RNA ein Polynukleotid enthält, das Basensequenzen einschließt, die komplementär zu Basensequenzen von einer Base stromaufwärts bis 20 Basen bis 24 Basen stromaufwärts der PAM-Sequenz in den Zielgenen sind.

## Revendications

1. Cellules reproductrices femelles dérivées d'un mammifère non-humain génétiquement modifié **caractérisées en ce qu'**une protéine ou un ARNm de Cas9 d'origine maternelle s'accumule dans les cellules reproductrices femelles, dans lesquelles le mammifère non-humain génétiquement modifié présente un génome nucléaire dans lequel au moins 3 copies de gènes qui codent pour Cas9 (9 associé à CRISPR) sont introduites et dans lesquelles le mammifère non-humain génétiquement modifié est un sélectionné dans le groupe constitué d'une souris, d'un rat, d'un hamster, d'un cochon d'Inde, d'un lapin, d'un chien, d'un chat, d'un cheval, d'une vache, d'un mouton, d'un porc, d'une chèvre, d'un ouistiti et d'un singe.

2. Cellules reproductrices femelles selon la revendication 1, dans lesquelles le noyau des cellules reproductrices femelles ne contient pas les gènes qui codent pour la Cas9.

3. Cellules reproductrices femelles selon la revendication 1 ou 2, dans lesquelles la Cas9 est un variant.

4. Cellules reproductrices femelles selon la revendication 3, dans lesquelles la Cas9 est un variant dépourvu d'activité de clivage d'un ou des deux brins d'un gène cible contenant une séquence cible.

5. Cellules reproductrices femelles selon l'une quelconque des revendications 1 à 4, dans lesquelles les gènes qui codent pour la Cas9 sont introduits en tandem dans le génome nucléaire.

6. Cellules reproductrices femelles selon l'une quelconque des revendications 1 à 5, dans lesquelles le mammifère non-humain génétiquement modifié est un sélectionné dans le groupe constitué d'une souris, d'un rat, d'un hamster, d'un cochon d'Inde, d'un lapin, d'un chien, d'un chat, d'un cheval, d'une vache, d'un mouton, d'un porc, et d'une chèvre.

7. Procédé de modification de gènes cibles, comprenant une étape d'introduction d'un ARN guide dans des cellules reproductrices femelles selon l'une quelconque des revendications 1 à 6.

8. Procédé de modification de gènes cibles, comprenant :
- une étape d'introduction d'un ARN guide dans des cellules reproductrices femelles selon l'une quelconque des revendications 1 à 3, 5 ou 6,
- une étape de clivage des gènes cibles au niveau d'un site de clivage situé en amont d'une séquence PAM (motif de reconnaissance du proto-espaceur) par une Cas9 exprimée dans les cellules reproductrices femelles, et
- une étape d'obtention des gènes cibles modifiés dans une région déterminée par une liaison complémentaire de l'ARN guide et des gènes cibles,
dans lequel les gènes cibles contiennent la séquence PAM, et l'ARN guide contient un polynucléotide comprenant une séquence de bases complémentaire à une séquence de bases en amont de la séquence PAM dans les gènes cibles.

9. Procédé de modification de gènes cibles, comprenant :
- une étape d'introduction d'un ARN guide dans des cellules reproductrices femelles selon l'une quelconque des revendications 1 à 3, 5 ou 6,
- une étape de clivage des gènes cibles au niveau d'un site de clivage situé trois bases en amont d'une séquence PAM par une Cas9 exprimée dans les cellules reproductrices femelles, et
- une étape d'obtention des gènes cibles modifiés dans une région déterminée par une liaison complémentaire de l'ARN guide et des gènes cibles,
dans lequel les gènes cibles contiennent la séquence PAM comprenant NGG (N est n'importe quelle base sélectionnée dans le groupe constitué de l'adénine, de la cytosine, de la thymine, et de la guanine), et l'ARN guide contient un polynucléotide comprenant des séquences de bases complémentaires à des séquences de bases allant d'une base en amont à 20 bases à 24 bases en amont de la séquence PAM dans les gènes cibles.
